# EUROPEAN PATENT SPECIFICATION

(11) **EP 3 765 434 B1**
(45) Date of publication and mention of the grant of the patent: **27.11.2024**
(21) Application number: 19719939.1
(22) Date of filing: 08.03.2019
(51) Int. Cl.: C07C 37/50, C07C 39/04, C07C 39/06, C07C 41/01, C07C 43/23, C07C 2/86

(54) **CATALYTIC CONVERSION OF BIOMASS TO BIOPHENOL**
KATALYTISCHE UMWANDLUNG VON BIOMASSE IN BIOPHENOL
CONVERSION CATALYTIQUE DE BIOMASSE EN BIOPHÉNOL

(30) Priority: 15.03.2018 NL 2020595
(43) Date of publication of application: 20.01.2021
(73) Proprietor: Vertoro B.V., 6167 RD Geleen (NL)
(72) Inventor: HENSEN, Emiel Jan Maria, 5612 AZ Eindhoven (NL); HUANG, Xiaoming, 5612 AZ Eindhoven (NL); OUYANG, Xianhong, 5612 AZ Eindhoven (NL)
(74) Representative: IPecunia
(86) International application number: PCT/NL2019/050151
(87) International publication number: WO 2019/177458

(56) References cited:
- WO-A1-2014/168473
- WO-A1-2016/187678
- GB-A- 1 079 147
- US-A- 4 230 896
- XIAOMING HUANG ET AL: "Selective Production of Biobased Phenol from Lignocellulose-Derived Alkylmethoxyphenols", ACS CATALYSIS, vol. 8, no. 12, 22 October 2018 (2018-10-22), US, pages 11184 - 11190, XP055598299, ISSN: 2155-5435, DOI: 10.1021/acscatal.8b03430
- DANNY VERBOEKEND ET AL: "Alkylphenols to phenol and olefins by zeolite catalysis: a pathway to valorize raw and fossilized lignocellulose", GREEN CHEMISTRY, vol. 18, no. 1, 1 January 2016 (2016-01-01), GB, pages 297 - 306, XP055520161, ISSN: 1463-9262, DOI: 10.1039/C5GC01868D
- TAKUYA YOSHIKAWA ET AL: "Conversion of alkylphenol to phenol via transalkylation using zeolite catalysts", CATALYSIS TODAY, 1 August 2018 (2018-08-01), AMSTERDAM, NL, XP055520213, ISSN: 0920-5861, DOI: 10.1016/j.cattod.2018.08.009

## Description

The present invention relates to the field of catalytic conversion of biomass to biophenol. More particularly, the invention relates to a method for demethoxylation and/or transalkylation of lignin-derived products and methods for obtaining phenol from lignin-derived alkylmethoxyphenols via a tandem demethoxylation and transalkylation processes.

Phenol is a commercially available product and an important chemical intermediate with a global annual production of 10.7 million tonnes in 2017 (cf. 8.9 million tonnes in 2012). The Asian-Pacific region is the largest producer of phenol. The dominant manufacturing process is the cumene process. Phenol is predominantly converted to precursors for plastics. For instance, bisphenol-A is a precursor to polycarbonates and epoxide resins. Other plastics are phenolic resins. Hydrogenation of phenol to cyclohexanone is a route to nylon production. An important class of products are non-ionic detergents.

Approximately, 98 percent of all phenol is produced by the cumene process, which also accounts for 20 percent of the global benzene demand. However, this process depends on fossil based feedstock which is not sustainable.

Accordingly, many industrial applications can benefit from the development of a process to obtain phenol from a renewable feedstock. Phenol is a drop-in biobased intermediate and as such it is imperative that the cost should not exceed that of fossil-based phenol. On the other hand, the (fossil-based) cumene process is a three-step process with a large environmental burden. Moreover, it co-produces equimolar amounts of acetone which decreases the economic viability when acetone demand is too low.

An efficient and green process for the production of phenol based on renewable biomass is lacking. A possible solution for selective production of biophenol is the use of the lignin fraction of lignocellulosic biomass.

Alkylmethoxyphenols (e.g. 2-methoxy-4-propylphenol) can be selectively produced from lignocellulosic biomass in good-to-excellent yield via "reductive fractionation" techniques which comprise catalytic hydrogenolysis of lignin fragments solvolytically released from whole biomass in alcohol solvents. For instance, Song et al. studied valorization of birch wood lignin into alkylmethoxyphenols in alcohols over a nickel catalyst [Energy Environ. Sci. 2013, 6, 994]. Abu-Omar *et al.* discussed a bimetallic Zn/Pd/C catalyst that directly converts lignin from biomass into two alkylmethoxyphenols in methanol [Green Chem. 2015, 17, 1492]. Sels *et al.* demonstrated delignification of birch wood in methanol using Ru/C or Pd/C, resulting in a limited number of alkylmethoxyphenols and carbohydrate pulp [Energy Environ. Sci. 2015, 8, 1748; Chem. Comm. 2015, 51, 13158]. From these processes, mainly 2-methoxy-4-propylphenol and 2,6-dimethoxy-4-propylphenol could be produced in high yield.

There are many known lignin depolymerization processes. Different processes deliver different products, but the general structure of the products are similar, which are phenolic compounds substituted by methoxy and alkyl groups. The "reductive fractionation" approach which is also known as "lignin-first" is a well-known technology, this process is able to selectively produce alkylmethoxyphenols in high yield.

The alkylmethoxyphenols largely depends on the source of wood and the process that were used for lignin depolymerization. If hardwood (e.g. birch, poplar, eucalyptus) is used as feedstock, the main products are 4-n-propylsyringol (PS-H), 4-n-propanolsyringol (PS-OH) and 4-n-methoxy propyl syringol (PS-OCH₃). When softwood (e.g. pine, Douglas fir) is used as feedstock, the main products are 4-n-propylguaiacol (PG-H), 4-n-propanolguaiacol (PG-OH) and 4-n-methoxy propyl guaiacol (PG-OCH₃). By changing the catalyst and reaction conditions, it is possible to control the product distributions of the depolymerization process.

The lignin oils obtained from depolymerization are typically composed of a wide range of methoxylated alkylphenols which have limited applications.

Hydrodeoxygenation (HDO) of lignin-derived oil and model compounds (e.g. guaiacol) have been extensively studied. Most of these techniques resulted in formation of oxygen-free and ring-saturated products and suffered from catalyst deactivation. Besides, these techniques do not use benzene or an aromatic as a solvent. Methoxy groups are removed either by forming small molecules such as methane or methanol or by forming methylated phenols via intramolecular methyl transfer reaction. The formation of CH₄ or methanol results in decreasing the carbon atomic efficiency of the process. The formation of methylated phenols also results in reducing the yield of phenol as the demethylation has been proven very difficult. Selective removal of the methoxy group while keeping the phenolic hydroxyl group and aromatic ring intact is challenging. Besides, in earlier research guaiacol was usually chosen as the model compound, which is less representative because it is free of alkyl side chains. Selective hydrodeoxygenation of guaiacol to produce phenol and alkylphenol has for example been described previously in ACS Catal., 2017, 7, 695-705.

Dealkylation and transalkylation of alkylbenzenes is well-known catalytic chemistry in the petrochemical industry. However, detailed knowledge about dealkylation of alkylphenols is quite limited. One significant challenge is coke formation due to the polymerization of olefins (the alkyl side-groups are usually removed as olefins), which leads to catalyst deactivation. Hydrogen is typically added to the reaction mixture to convert olefins into less reactive paraffins in order to reduce the coke formation. However, paraffins are less valuable than olefins. Another undesired side-reaction is the removal of the phenolic hydroxyl group which leads to the formation of an alkylbenzenes and water. Alkylbenzenes are also interesting chemical intermediates, but less valuable than phenol. Catalytic steam dealkylation (SDA) has also been applied instead of hydrodealkylation (HDA). One most relevant prior art reported the use of zeolites catalyst such as HZSM-5 to remove the propyl group from n-propylphenol by co-feeding with the steam (Reaction 1). This process produces phenol and propylene as the main products. [Green Chem., 2016, 18, 297-306.].

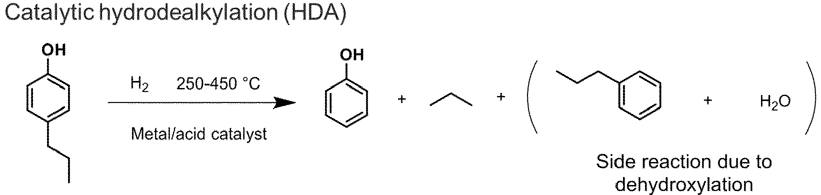

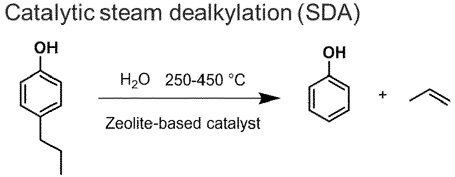

The dealkylation process described by Verboekend et al. in Green Chem., 2016, 18, 297-306 (Reaction 1) is performed at atmospheric pressure without the presence of hydrogen. However performing this reaction at low pressure in the absence of hydrogen results in quick catalyst deactivation. Verboekend et al however do not describe a demethoxylation process, therefore the process described is only suitable for n-propylphenol.

GB 1079147 describes a process for thermal hydrodealkylation of an alkyl hydroxyl aromatic compound such as cresols, to obtain a hydroxyl aromatic, such as phenol. This process do not use any catalyst, therefore, there is no catalysis in this process. GB 1079147 describes the use of alkyl aromatics in the process. A disadvantage of the process described is the high temperature required, of 482 to 871 °C. Such high temperatures would make the process not competitive with using fossil fuel as a feed stock, and is therefore undesirable. GB 1079147 does also not describe a demethoxylation process.

WO 2016/187678 describes a process for the production of 3-alkylphenols and uses thereof.

WO 2014/168473 describes a method for the depolymerization of lignin.

US 4 230 896 describes a catalytic steam dealkylation of alkyl phenols.

There are multiple examples of alternative methods for dealkylation such as thermal cracking, catalytic hydrodealkylation (HDA) and catalytic steam dealkylation (SDA). However, these processes either involve the use of very high temperature (600-700°C), or the catalyst suffers from deactivation due to coke formation. A second drawback of the HDA process is the unwanted dehydroxylation of some of the compounds.

These drawbacks, amongst others, are overcome by the novel process as detailed in the appended claims.

The present invention uses an aromatic as a solvent in the demethoxylation and transalkylation steps, combined with a catalyst for each reaction, thus enabling the formation of phenol through the transfer of the alkyl groups (both of the methoxy group and propyl side chain) to the aromatic compound. The resulting process has several advantages over the art, amongst others it does not result in dehydroxylation or coke formation (and catalyst deactivation), moreover the resulting alkylated aromatic compounds are more valuable when compared to paraffins. Further the demethoxylation step according to the invention can be performed together with the dealkylation step according to the invention in a single reactor, making the process less burdensome than the cumene process which involves three steps. Combining these two steps in a single reaction leads to higher productivity, is more energy efficient and results in lower capital cost.

### Summary of the invention.

The invention relates to a process for converting alkylmethoxyphenols, wherein alkylmethoxyphenols are demethoxylated in the presence of an aromatic solvent and a first catalyst to form alkylphenols, wherein the first catalyst contains a metal selected from groups consisting of Group 5 (VB), Group 6 (VIB), Group 7 (VI IB), Group 8 (VIIIB), Group 9 (VIIIB), Group 10 (VIIIB), Group 11 (IB), Group 12 (IIB), Group 13 (IIIA), Group 14 (IVA), Group 15 (VA) and Group 16 (VIA), wherein the IUPAC group number appears first and an old system of group numbers appears in parenthesis, or any alloy or mixture thereof, combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon oxide, wherein the aromatic solvents are selected from the group consisting of benzene, toluene, ethylbenzene, propylbenzene or combinations thereof.

The invention also relates to a process for converting alkylphenols, wherein alkylphenols are dealkylated in the presence of an aromatic solvent and a second catalyst to form phenol, wherein the second catalyst is a zeolite or zeotype material, wherein the aromatic solvents are selected from the group consisting of benzene, toluene, ethylbenzene, propylbenzene or combinations thereof.

In an embodiment of the invention the process comprises a demethoxylation process according to the invention and a dealkylation process according to the invention.

In a further embodiment of the invention the demethoxylation process and the dealkylation process take place in the same reactor.

In a further embodiment of the invention the processes are performed in a continuous fixed bed flow reactor or a batch reactor.

In a further embodiment of the invention the demethoxylation and/or the dealkylation process is performed at a temperature between 200 to 500 °C, preferably 250 - 450 °C, more preferably 300 - 400 °C most preferably around 350 °C.

In a further embodiment of the invention the demethoxylation and/or the dealkylation process is performed at a pressure of 1 - 250 bar, preferably 10 - 200 bar, more preferably 50 - 180 bar, most preferably 80 - 150 bar.

In a further embodiment of the invention the demethoxylation and/or the dealkylation process is performed such that the residence time in the reactor is between 0.5 and 24 hours, preferably between 1 and 18 hours, more preferably between 2 and 12 hours, even more preferably between 4 and 10 hours, most preferably at least 2 hours.

In a further embodiment of the invention the aromatic solvent is at least 50% v/v benzene, more preferably at least 80% v/v benzene, even more preferably at least 90% v/v benzene.

In a preferred embodiment the zeolite or zeotype material is characterized by:
(i) acid sites of sufficient strength, preferably being aluminosilicates or silicates with metal and/or metalloid substitutions, more preferably silicates with Fe, Ga and/or B substitutions, and
(ii) having a pore structure ranging from 3 - 13 Angstrom, preferable having a pore structure with ranges identified for 10 membered ring zeolites.

In a further embodiment of the invention the second catalyst used in the dealkylation process comprises aluminosilicates, preferably the second catalyst is HZSM-5.

In a further embodiment of the invention the first catalyst is Au/TiO₂.

In a further embodiment of the invention the amount of metal loaded on the support of the first catalyst is in the range of 0.1 - 30%, preferably 0.5 - 20%, more preferably 1 - 10%.

In a further embodiment of the invention the first catalyst is a SiO₂ supported metal phosphide catalyst.

In a further embodiment of the invention the first catalyst is selected from the group consisting of CoP/SiO₂, WP/SiO₂ and MoP/SiO₂.

In a preferred embodiment the first catalyst is MoP/SiO₂.

In a preferred embodiment the first catalyst is MoP/SiO₂ and the catalyst has a Mo loading of 1.6 mmol/g or less, preferably 1.2 mmol/g or less, more preferably 0.8 mmol/g or less, even more preferably between 0.1 and 0.8 mmol/g, most preferably between 0.4 and 0.8 mmol/g.

In a further embodiment of the invention the first and the second catalyst are either stacked or homogeneously mixed with each other.

In a further embodiment of the invention the demethoxylation and/or the dealkylation process are performed in an inert atmosphere, preferably the inert atmosphere is gas, more preferably it is air, nitrogen, hydrogen or a combination of thereof, most preferably the inert atmosphere is hydrogen.

In a further embodiment of the invention the alkylmethoxyphenols and/or the alkylphenols are lignin-derived, preferably the alkylmethoxyphenols are obtained from depolymerisation of lignin.

In an alternative embodiment, the invention relates to a dealkylation process for dealkylating alkylphenols, wherein an aromatic solvent and a second catalyst are used to generate phenol and alkylated aromatics.

In an embodiment of the invention, the reaction is preceded by a demethoxylation process, wherein alkylmethoxyphenols are demethoxylated in the presence of a first catalyst to form the alkylphenols to be used in the dealkylation process. Preferably, the dealkylation process and the demethoxylation process take place in the same reactor. More preferably the processes are performed in a continuous fixed bed flow reactor or a batch reactor.

In a preferred embodiment of the invention the dealkylation and/or the demethoxylation process is performed at a temperature between 200 to 500 °C, preferably 250 - 450 °C, more preferably 300 - 400 °C most preferably around 350 °C. In a preferred embodiment of the invention the dealkylation and/or the demethoxylation process is performed at a pressure of 1 - 200 bar, preferably 10 - 150 bar, more preferably 50 - 100 bar.

In a preferred embodiment of the invention the dealkylation and/or the demethoxylation process is performed such that the residence time in the reactor is between 0.5 and 24 hours, preferably between 1 and 18 hours, more preferably between 2 and 12 hours, even more preferably between 4 and 10 hours, most preferably at least 2 hours.

In a preferred embodiment of the invention wherein the aromatic solvents are selected from the group consisting of benzene, toluene, ethylbenzene, propylbenzene, xylenes, or combinations thereof, in an even more preferred embodiment the aromatic solvent is benzene.

In a preferred embodiment of the invention the second catalyst used in the dealkylation process is a zeolite or zeotype material. In a more preferred embodiment the zeolite or zeotype material is characterized by:
(i) acid sites of sufficient strength, preferably being aluminosilicates or silicates with metal and/or metalloid substitutions, more preferably silicates with Fe, Ga and/or B substitutions, and
(ii) having a proper pore structure ranging from 3-13 Angstrom, preferable having a proper pore structure with ranges identified for 10 membered ring zeolites.

In an even more preferred embodiment the second catalyst used in the dealkylation process comprises aluminosilicates, most preferably the second catalyst is HZSM-5.

In a further embodiment of the invention the first catalyst used in the demethoxylation process contains a metal selected from groups consisting of Group 5 (VB), Group 6 (VIB), Group 7 (VIIB), Group 8 (VIIIB), Group 9 (VIIIB), Group 10 (VIIIB), Group 11 (IB), Group 12 (IIB), Group 13 (IIIA), Group 14 (IVA), Group 15 (VA) and Group 16 (VIA), wherein the IUPAC group number appears first and an old system of group numbers appears in parenthesis, or any alloy or mixture thereof, combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon oxide.

In a more preferred embodiment the first catalyst is Au/TiO₂.

In a preferred embodiment of the invention the amount of metal loaded on the support of the second catalyst is in the range of 0.1 - 30%, preferably 0.5 - 20%, more preferably 1 - 10%.
In a more preferred embodiment of the invention the first and the second catalyst are either stacked or homogeneously mixed with each other.

In a preferred embodiment of the invention the dealkylation and/or the demethoxylation process are performed in an inert atmosphere, preferably the inert atmosphere is gas, more preferably it is air, nitrogen, hydrogen or a combination of thereof.

In a preferred embodiment of the invention the alkylmethoxyphenols and/or the alkylphenols are lignin-derived, preferably the alkylmethoxyphenols are obtained from depolymerisation of lignin.

### Detailed description of the invention

The present invention describes a technology that is able to selectively convert lignin derived alkylmethoxyphenols into phenol by coupling the demethoxylation and transalkylation reactions using benzene as a solvent. The demethoxylation can be catalyzed using a supported gold catalyst in the presence of hydrogen, yielding alkylphenols as the main products. Alternatively, metal phosphides can be used as an improved catalyst to catalyse the demethoxylation reaction. These alkylphenols can be dealkylated to form phenol catalyzed by the 8-, 10-, and 12-membered ring zeolite such as HZSM-5, Mordenite and Y zeolite (HY). The alkyl groups are transferred to aromatic solvent such as benzene, toluene, xylenes or mixtures thereof, producing alkyl aromatics as the main products. A schematic overview of this process is presented in Reaction 2. The demethoxylation and dealkylation reactions can either be performed separately or combined together using a fixed-bed tubular reactor containing Au/TiO₂ and HZSM-5 catalysts.

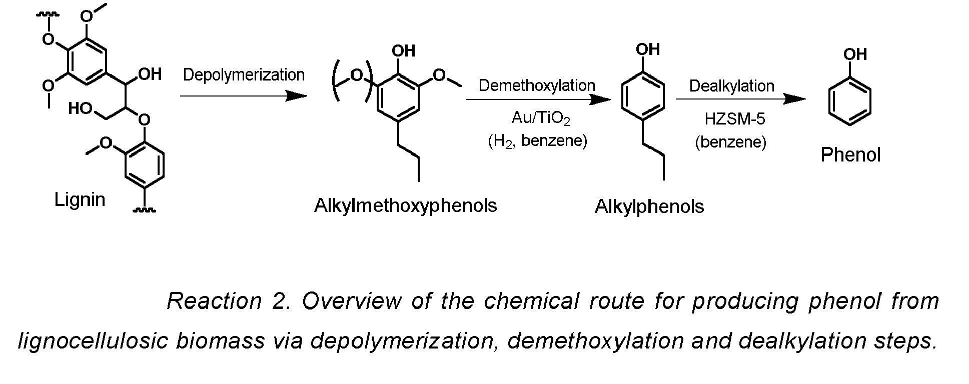

Therefore in a first aspect of the invention the invention relates to a process for converting alkylmethoxyphenols, wherein alkylmethoxyphenols are demethoxylated in the presence of an aromatic solvent and a first catalyst to form alkylphenols, wherein the first catalyst contains a metal selected from groups consisting of Group 5 (VB), Group 6 (VIB), Group 7 (VI IB), Group 8 (VIIIB), Group 9 (VIIIB), Group 10 (VIIIB), Group 11 (IB), Group 12 (IIB), Group 13 (IIIA), Group 14 (IVA), Group 15 (VA) and Group 16 (VIA), wherein the IUPAC group number appears first and an old system of group numbers appears in parenthesis, or any alloy or mixture thereof, combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon oxide, wherein the aromatic solvents are selected from the group consisting of benzene, toluene, ethylbenzene, propylbenzene or combinations thereof.

In a second aspect the invention relates to a process for converting alkylphenols, wherein alkylphenols are dealkylated in the presence of an aromatic solvent and a second catalyst to form phenol, wherein the second catalyst is a zeolite or zeotype material, wherein the aromatic solvents are selected from the group consisting of benzene, toluene, ethylbenzene, propylbenzene or combinations thereof.

It has now surprisingly been found by the inventors that both the demethoxylation process and the dealkylation (transalkylation) process independently benefit from the presence of an aromatic solvent, such as for example benzene, toluene, ethylbenzene or propyl benzene (n-propyl benzene). The advantage of using an aromatic solvent in the in the demethoxylation process is that efficiency of the process is improved. Further by transferring the methyl group to the aromatic solvent more valuable toluene and xylenes are formed, thereby also significantly reducing the formation of undesired methylated phenols. The advantage of using an aromatic solvent in the in the dealkylation (transalkylation) process is that also the efficiency of the process is improved. Further using a transalkylation step in an aromatic solvent results in higher value products such as cumene and n-propylbenzene. Lastly, by using an aromatic solvent the disadvantage of coke formation and subsequent catalyst inactivation is surprisingly avoided.

An additional advantage of using an aromatic solvent for either the demethoxylation process or the dealkylation (transalkylation) process that it surprisingly renders the demethoxylation process suitable to be combined with the dealkylation process and *vice versa.*

In a further embodiment the invention relates to a process, comprising the demethoxylation process according to the invention and further comprising the dealkylation process according to the invention.

It has now been found that by using an aromatic solvent the demethoxylation process and the dealkylation process can efficiently be combined in a single process, thereby greatly increasing the energy efficiency of the overall process and increasing the conversion rate and yield, while reducing capital cost to conduct the process. A process where the demethoxylation and dealkylation step are combined can be performed in a single reactor, the process may be a batch reaction or a continuous fixed bed flow reactor, wherein the catalysts for the demethoxylation and the dealkylation step can either be mixed or stacked.

Alternatively, the invention relates a process for converting alkylmethoxyphenols, wherein alkylphenols are dealkylated in the presence of an aromatic solvent and a second catalyst are used to generate phenol and alkylated aromatics.

Preferably the alkylphenols that are dealkylated according to the invention are alkylmethoxyphenols, or are alkylmethoxyphenols that are demethoxylated to alkylphenols prior to the dealkylation process.

In a further alternative embodiment the invention relates to a process, wherein the dealkylation process is preceded by a demethoxylation process, wherein alkylmethoxyphenols are demethoxylated in the presence of a first catalyst to form the alkylphenols to be used in the dealkylation process. Preferably, the dealkylation process and the demethoxylation process take place in the same reactor. More preferably the processes are performed in a continuous fixed bed flow reactor or a batch reactor.

Preferably the process for converting alkylmethoxyphenols according to the invention is for converting lignin derived alkylmethoxyphenols. More preferably, the process for converting alkylmethoxyphenols is for converting alkylmethoxyphenols to phenol, and optionally alkylbenzenes.

Dealkylation, when used herein refers to the transfer of an alkyl group from alkylphenols to benzene, therefore the term dealkylation and transalkylation when used herein are used interchangeably.

The preferred starting material for this process is propylguaiacol. The "reductive fractionation" approach which is also known as "lignin-first" is compatible with our process, because this process is able to selectively produce alkylmethoxyphenols in high yield. Nevertheless, as long as the process delivers a product stream rich in alkylmethoxyphenols, it is compatible with our process.

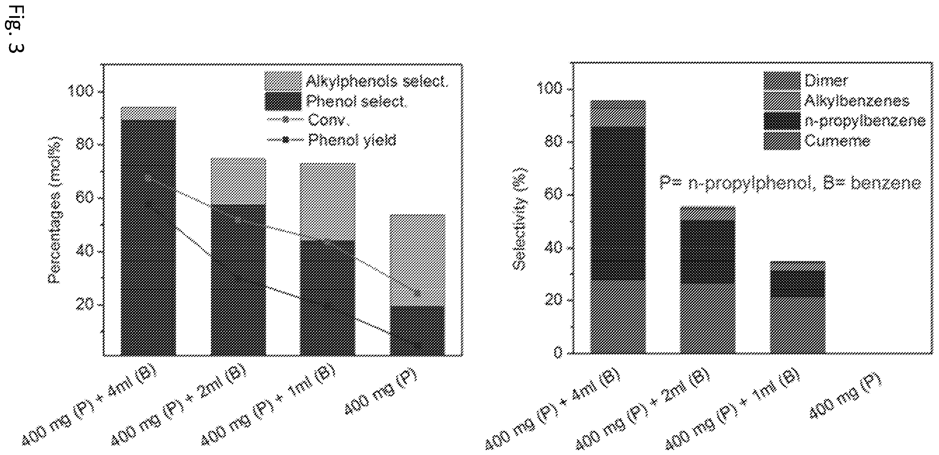

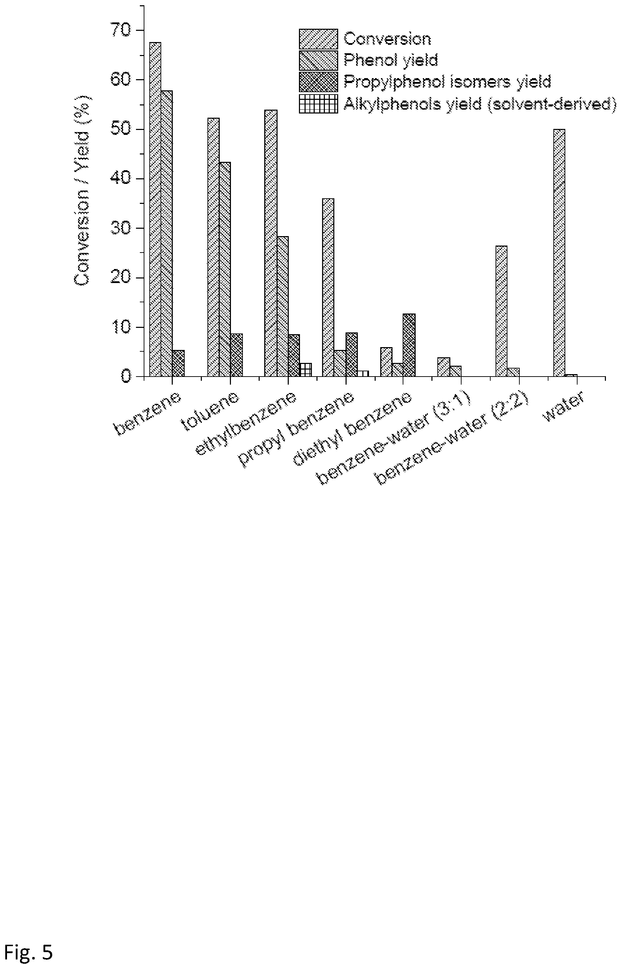

Contrary to processes in the art which describe the dealkylation of guaiacol, the process of the invention is based on authentic lignin-derived molecules such as (amongst others) 4-propylphenol and 2-methoxy-4-propylphenol, which makes this process stand out from others. Further, the process of the invention makes use of benzene or other aromatics as a solvent. Figure 3 demonstrates increased yield when benzene is used. Figure 5 demonstrates improved conversion and yield for several aromatic compounds, with the most profound improvements when benzene, toluene, ethylbenzene or propyl benzene are used.

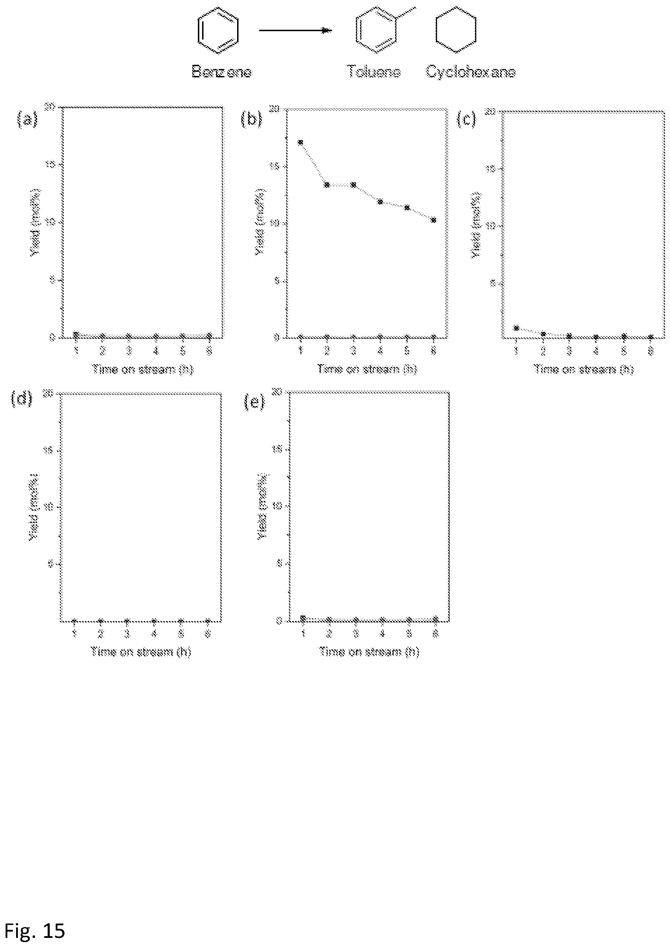

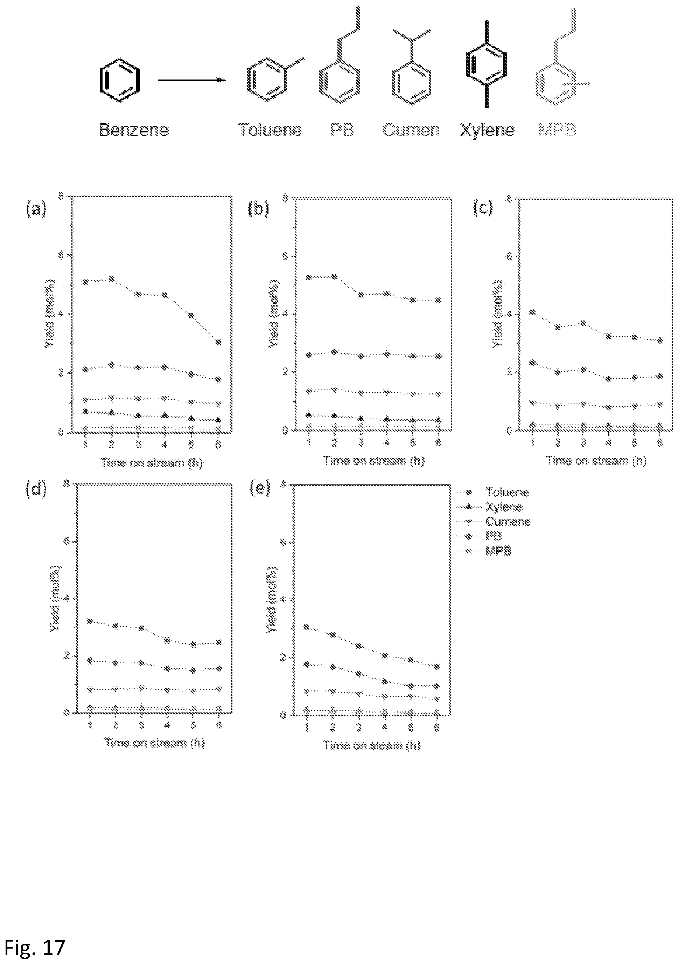

For the demethoxylation step, no ring-saturated products were produced and the phenolic hydroxyl group is retained. Moreover, instead of removing the methoxy groups as methane or methanol, the methyl group can be effectively transferred to an aromatic solvent by forming more valuable toluene and xylenes, thereby significantly reducing the formation of undesired methylated phenols and improving the carbon atomic efficiency of the process (Reaction 3, Figure 15 and 17).

For the dealkylation step, the alkyl groups of alkylphenols can be effectively transferred to benzene, yielding phenol as the main product. Moreover, the demethoxylation and transalkylation steps can be integrated in a single reactor using a fixed-bed reactor, thereby significantly reducing capital cost.

This invention describes a one-step process that is able to selectively convert lignin-derived alkylmethoxyphenols (e.g. 2-methoxy-4-propylphenol) into phenol in high yield by coupling the demethoxylation and transalkylation reactions in one reactor containing hydrogen, aromatic solvent such as benzene, toluene, xylenes or mixtures thereof, supported metal and zeolite catalysts. The novel dealkylation process that we disclose herein is based on the transalkylation of alkyl side-chains from alkylphenol to benzene and other aromatics. Specifically, we demonstrate that the alkyl group of the alkylphenols can be effectively transferred to benzene using a zeolite or zeotype catalyst such as HZSM-5, mordenite or HY (Reaction 3). The transalkylation of alkyl chains to the benzene results in formation of mainly cumene and n-propylbenzene which are higher value products. Moreover, cumene is also the starting material for phenol production.

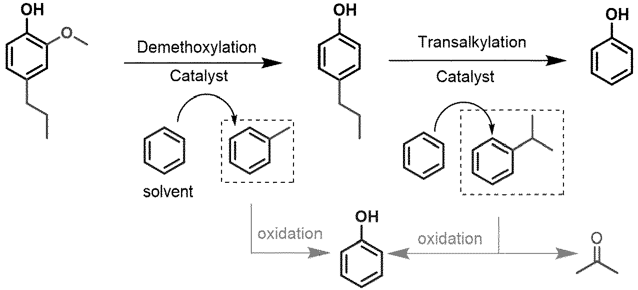

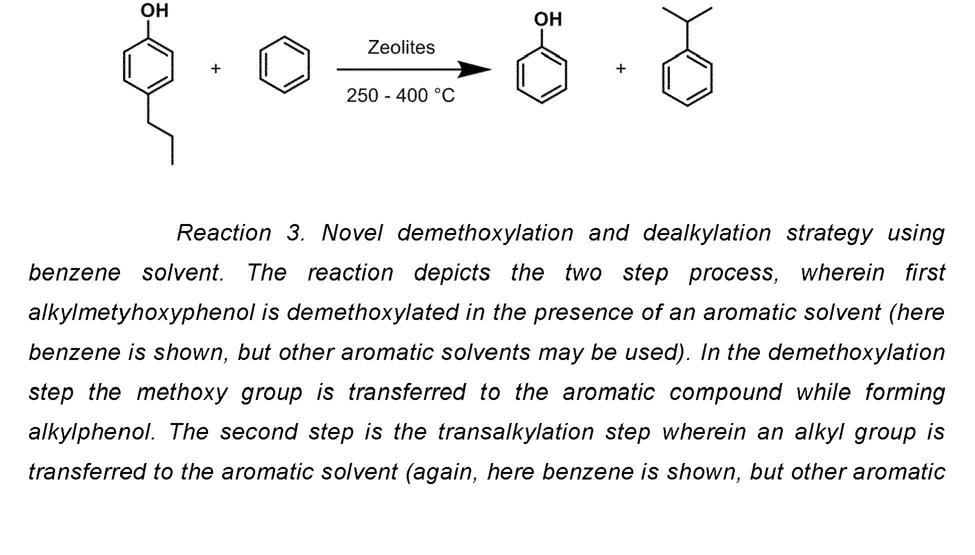

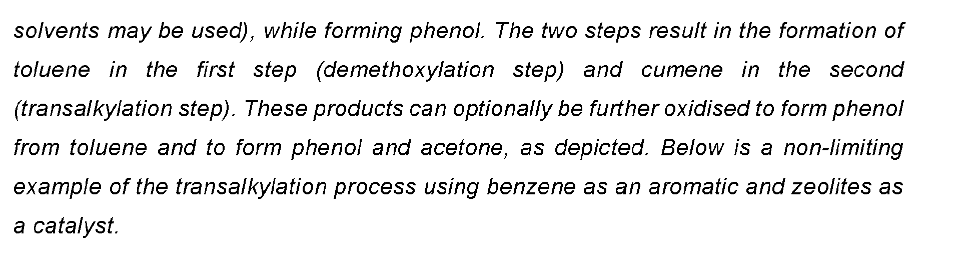

We found that this process can be easily integrated in a single step by combining a catalyst to carry out the demethoxylation step (selectively removing the methoxy group by for example a supported Au catalyst) with a catalyst to carry out the dealkylation step. Therefore in an embodiment the invention relates to a demethoxylation process and/or a dealkylation process, wherein the demethoxylation process and the dealkylation process take place in the same reactor. In a particularly preferred embodiment of the invention the processes are performed in a continuous fixed bed flow reactor or a batch reactor.

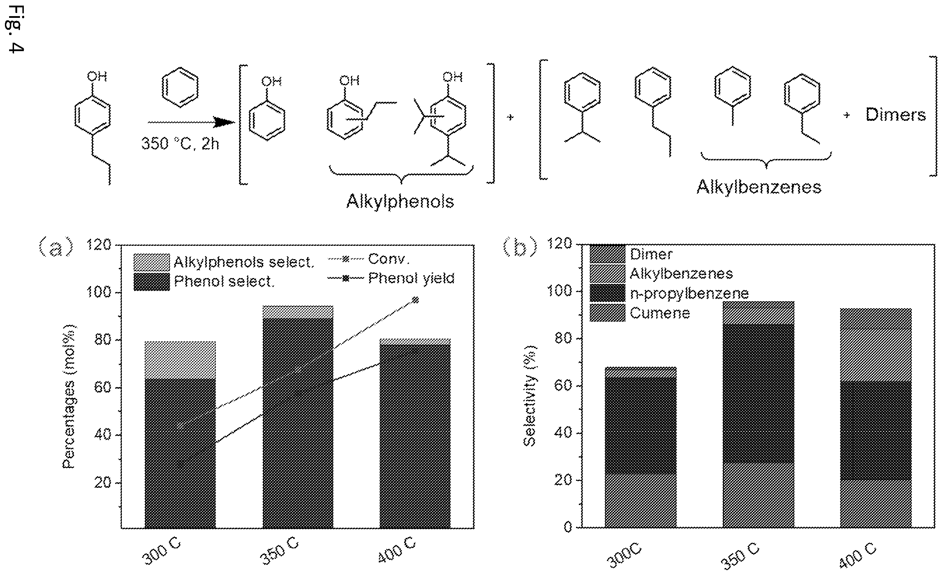

Figure 4 demonstrates the influence of the temperature on yield and conversion. Therefore, in a preferred embodiment of the invention the demethoxylation and/or the dealkylation process is performed at a temperature between 200 to 500 °C, preferably 250 - 450 °C, more preferably 300 - 400 °C most preferably around 350 °C.

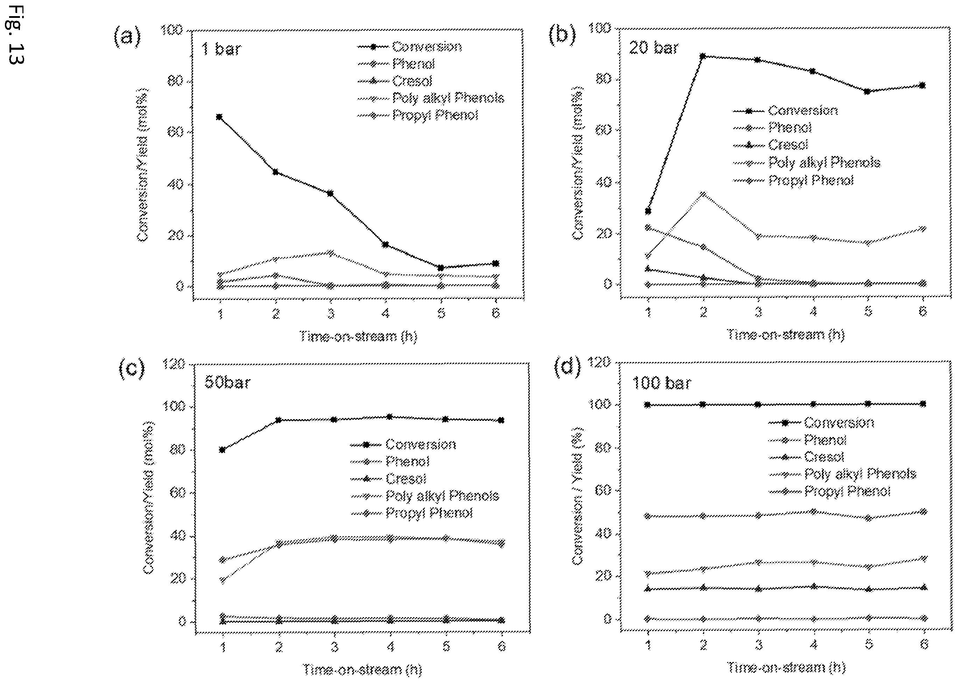

In a preferred embodiment the process according to the invention is performed at a pressure of 1 - 250 bar, preferably 10 - 200 bar, more preferably 50 - 180 bar, most preferably 80 - 150 bar. Figure 13 demonstrates increased conversion efficiency and yield by increasing the pressure, it is therefore understood that the demethoxylation process according to the invention is preferably performed at a pressure of 50 bar or higher, more preferably at 60 bar or higher, 70 bar or higher, 80 bar or higher, 90 bar or higher, or 100 bar or higher. It is further understood that the dealkylation (transalkylation) process according to the invention is preferably performed at a pressure of 50 bar or higher, more preferably at 60 bar or higher, 70 bar or higher, 80 bar or higher, 90 bar or higher, or 100 bar or higher. It is further noted that figure 13 demonstrates catalyst deactivation due to coke formation when low pressure such as 1 bar is used, as can be seen be the reduced conversion over time.

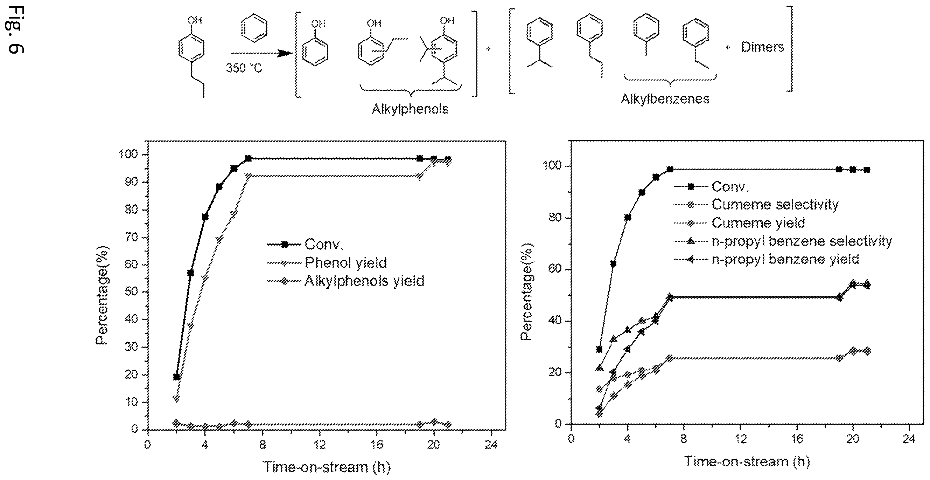

In a preferred embodiment the demethoxylation process is performed such that the residence time in the reactor is between 0.5 and 24 hours, preferably between 1 and 18 hours, more preferably between 2 and 12 hours, even more preferably between 4 and 10 hours, most preferably at least 2 hours. In a preferred embodiment the dealkylation process is performed such that the residence time in the reactor is between 0.5 and 24 hours, preferably between 1 and 18 hours, more preferably between 2 and 12 hours, even more preferably between 4 and 10 hours, most preferably at least 2 hours. In a preferred embodiment the combined demethoxylation and dealkylation process is performed such that the residence time in the reactor is between 0.5 and 24 hours, preferably between 1 and 18 hours, more preferably between 2 and 12 hours, even more preferably between 4 and 10 hours, most preferably at least 2 hours. Figure 6 demonstrates that maximum conversion and yield are generally achieved between 4 to 8 hours on stream, therefore in a particularly preferred embodiment the residence time in the reactor for either the demethoxylation process, or the dealkylation process or the combined demethoxylation and dealkylation process is 4 hours or more, preferably 5 hours or more, 6 hours or more, 7 hours or more or 8 hours or more, for example between 4 to 14 hours, 5 to 13 hours, 6 to 12 hours 7 to 11 hours or between 8 to 10 hours.

Figure 5 shows data for conversion rate and yield for different aromatics that can be used in the process according to the invention. The aromatic solvents for the demethoxylation process according to the invention are selected from the group consisting of benzene, toluene, ethylbenzene, propylbenzene, xylenes, or combinations thereof. In a preferred embodiment the aromatic solvent in the demethoxylation process is benzene. The aromatic solvents for the dealkylation process according to the invention are selected from the group consisting of benzene, toluene, ethylbenzene, propylbenzene, xylenes, or combinations thereof. In a preferred embodiment the aromatic solvent in the dealkylation process is benzene. Therefore in a preferred embodiment of the combined demethoxylation and dealkylation process according to the invention the aromatic solvents are selected from the group consisting of benzene, toluene, ethylbenzene, propylbenzene, xylenes, or combinations thereof. In a more preferred embodiment the aromatic solvent in the combined demethoxylation and dealkylation is benzene. It is to be understood that aromatic solvents like benzene are generally not 100% pure, moreover mixtures of different aromatic solvents can be used. In a preferred embodiment, the demethoxylation process, the dealkylation process or the combined demethoxylation and dealkylation process the aromatic solvent of the invention comprises benzene, toluene, ethylbenzene, propylbenzene, xylenes, or combinations thereof. Therefore in a preferred embodiment of the demethoxylation process, the dealkylation process or the combined demethoxylation and dealkylation process the aromatic solvent of the invention is or comprises at least 50% benzene, more preferably at least 80% benzene, even more preferably at least 90% benzene, even more preferably at least 95% most preferably 99% benzene. For example that aromatic solvent is benzene mixed with a different aromatic solvent or non-aromatic compound, wherein the mixture comprises 50%, 60%, 70%, 75%, 80%, 85%, 90%, 91%, 92%, 93%, 94%, 95%, 96%, 97%, 98% or 99% benzene.

Particularly good results were obtained by using zeolite or zeotype, such as HZSM-5, mordenite or Y zeolite, as a second catalyst in the process according to the invention. Therefore, the second catalyst used in the dealkylation process is a zeolite or zeotype material. More preferably the zeolite or zeotype material is characterized by:
(i) acid sites of sufficient strength, preferably being aluminosilicates or silicates with metal and/or metalloid substitutions, more preferably silicates with Fe, Ga and/or B substitutions, and
(ii) having a pore structure ranging from 3 - 13 Angstrom, preferable having a pore structure with ranges identified for 10 membered ring zeolites. In a particularly preferred embodiment the second catalyst used in the dealkylation process comprises aluminosilicates, preferably wherein the second catalyst is selected from HZSM-5, mordenite and Y zeolite, most preferably HZSM-5.

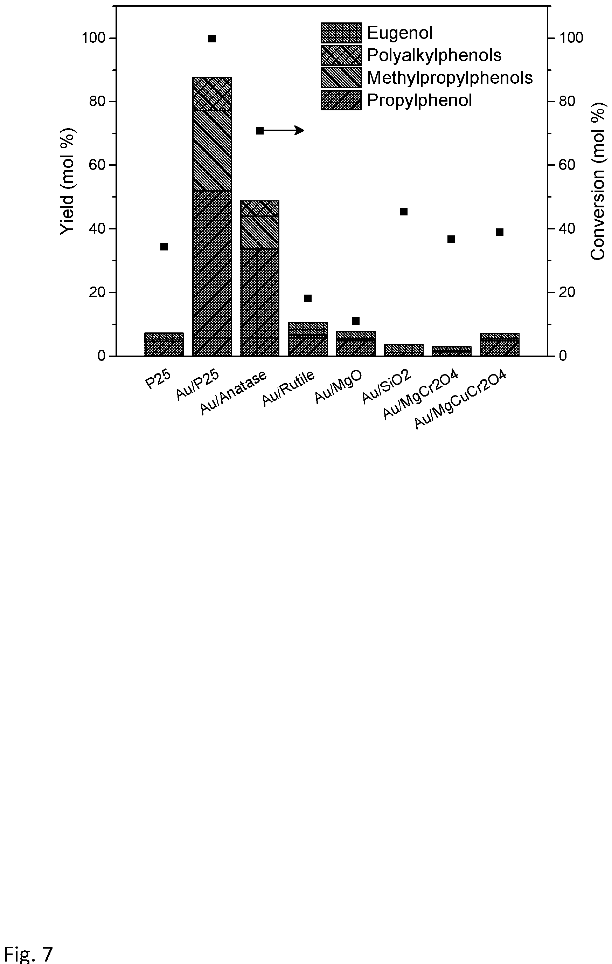

The novel demethoxylation process described herein selectively removes a methoxy group from an alkylmethoxyphenol compound in the presence of of a catalyst. Figure 7 demonstrates that higher yield and conversion can be achieved when using a combination of Au and TiO₂ (Au/P25 and Au/Anatase). Therefore, the demethoxylation process according to the invention, uses a first catalyst in the demethoxylation process which contains a metal selected from groups consisting of Group 5 (VB), Group 6 (VIB), Group 7 (VIIB), Group 8 (VIIIB), Group 9 (VIIIB), Group 10 (VIIIB), Group 11 (IB), Group 12 (IIB), Group 13 (IIIA), Group 14 (IVA), Group 15 (VA) and Group 16 (VIA), wherein the IUPAC group number appears first and an old system of group numbers appears in parenthesis, or any alloy or mixture thereof, combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon oxide. Preferably the first catalyst comprises gold combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon oxide. In a preferred embodiment of the invention the first catalyst is Au/TiO₂. In a preferred embodiment the amount of metal loaded on the support of the first catalyst is in the range of 0.1 - 30%, preferably 0.5 - 20%, more preferably 1 - 10%.

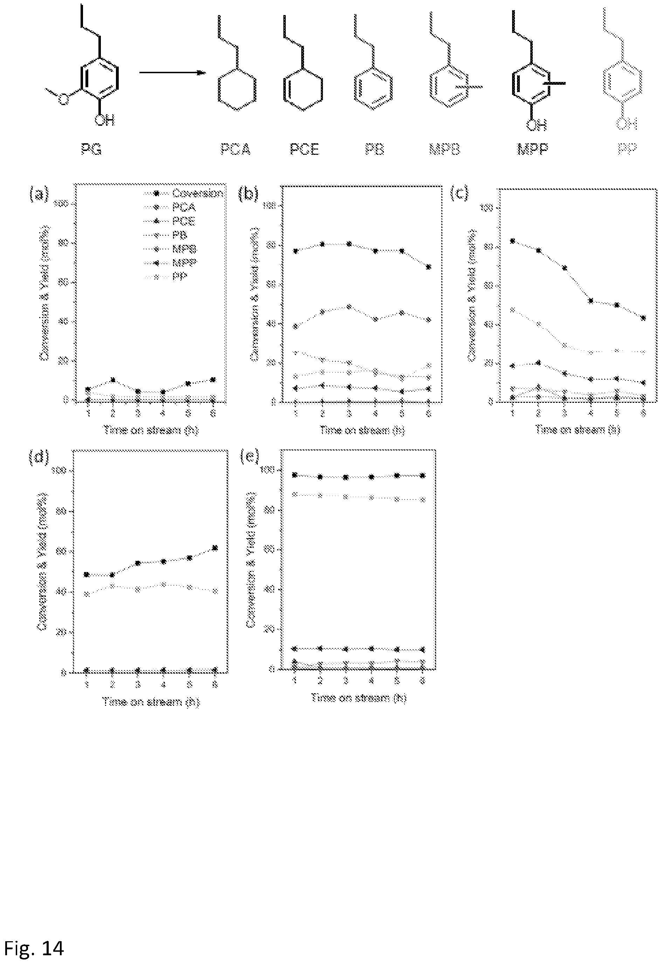

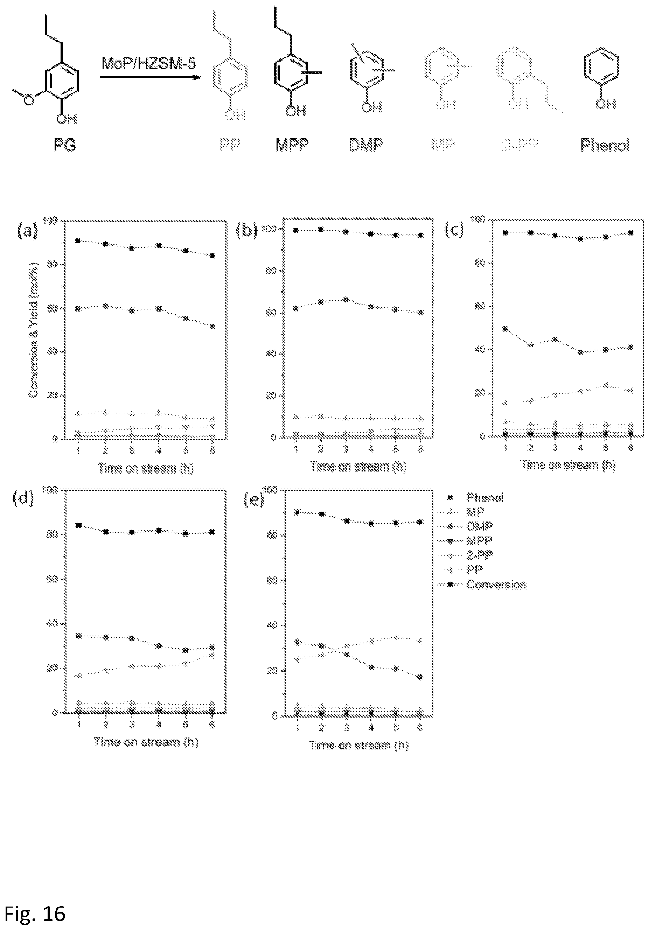

Even better results were obtained by using a SiO₂ supported metal phosphide catalyst to catalyse the demethoxylation reaction. The data presented in figures 14 and 16 demonstrate particularly high conversion and yield for such catalysts. Therefore, in a preferred embodiment of the invention the first catalyst is a SiO₂ supported metal phosphide catalyst. Preferably the first catalyst is selected from the group consisting of FeP/SiO₂, Ni₂P/SiO₂, CoP/SiO₂, WP/SiO₂ and MoP/SiO₂, or a combination thereof. More preferably the first catalyst is selected from the group consisting of CoP/SiO₂, WP/SiO₂ and MoP/SiO₂, or a combination thereof. Most preferably the first catalyst in the process according to the invention is MoP/SiO₂.

Therefore the demethoxylation step is preferably performed with a first catalyst wherein the first catalyst is either a supported metal as described herein, for example Au/TiO₂ or a SiO₂ supported phosphide, such as for example MoP/SiO₂.

Preferably, when using a SiO₂ supported metal phosphide catalyst as the first catalyst, the catalyst has a metal loading of 1.6 mmol/g or less, preferably 1.2 mmol/g or less, more preferably 0.8 mmol/g or less, even more preferably between 0.1 and 0.8 mmol/g, most preferably between 0.2 and 0.4 mmol/g. More preferably the first catalyst is MoP/SiO₂ and the catalyst has a Mo loading of 1.6 mmol/g or less, preferably 1.2 mmol/g or less, more preferably 0.8 mmol/g or less, even more preferably between 0.1 and 0.8 mmol/g, most preferably between 0.4 and 0.8 mmol/g.

A particular advantage of the process of the invention is that the demethoxylation and dealkylation step can be performed in a single reactor. Therefore, in a preferred embodiment of the invention the first and the second catalyst are either stacked or homogeneously mixed with each other.

In a preferred embodiment of the process of the invention, the demethoxylation and/or the dealkylation process are performed in an inert atmosphere, preferably the inert atmosphere is gas, more preferably it is air, nitrogen, hydrogen or a combination of thereof, most preferably the inert atmosphere is hydrogen.

The process of the invention is particularly useful to convert biomass such as lignin to phenol. Therefore in a preferred embodiment of the invention, the alkylmethoxyphenols and/or the alkylphenols are lignin-derived, preferably the alkylmethoxyphenols are obtained from depolymerisation of lignin. This process is useful because it provides an efficient bio-based alternative to phenol production from fossil fuel.

To the best of our knowledge, it is the most efficient way to selectively produce phenol from lignin-derived phenolic product streams. This can be achieved in a one-pot reaction approach. Another aspect is that in the transition from a fossil-based economy to a low-carbon economy in which bio-based feedstock also plays a role, integration of bio-based feedstock into fossil-based conversion processes in the chemical industry may be beneficial to increase the share of renewable drop-in chemicals and fuels.

### Description of the Figures

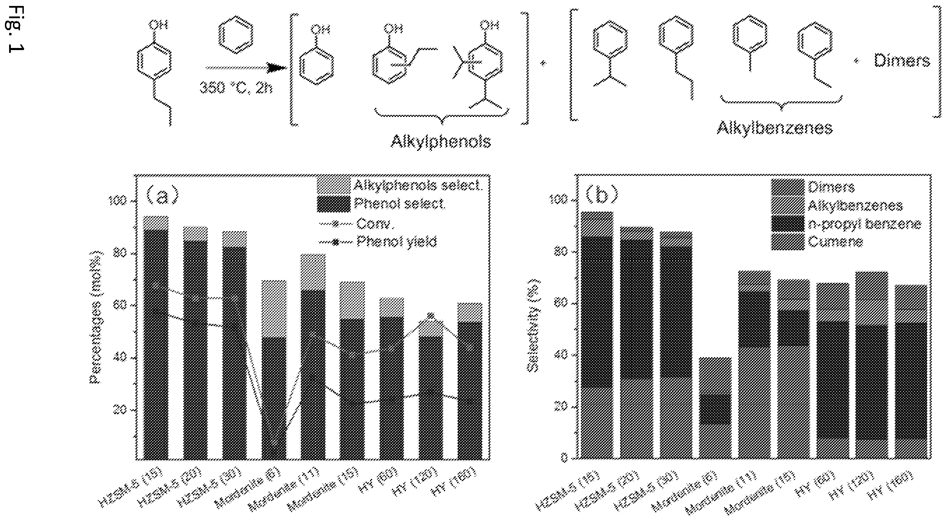

Figure 1. Product distribution of (a) n-propylphenol- and (b) benzene-derived product following dealkylation of n-propylphenol over the different catalysts in benzene solvent. (Conditions: 400 mg n-propylphenol, 4 ml benzene, 40 mg catalyst, 350 °C, 2h). Between brackets is indicated the Si/AI ratio of the HZSM-5 zeolite.

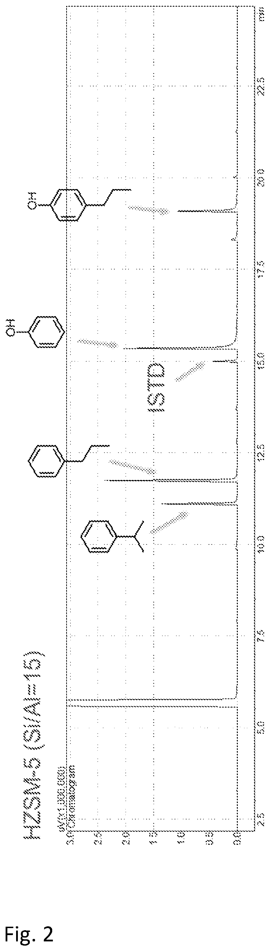

Figure 2. GC-MS chromatogram of the reaction mixture obtained from dealkylation of n-propylphenol at 350 °C for 2h over the HZSM-5 (Si/AI=15) in benzene solvent.
Figure 3. Influence of feedstock to benzene solvent ratio on product distributions over HZSM-5 (Si/AI=15) at 350 °C for 2h.
Figure 4. Influence of reaction temperature on product distributions over HZSM-5 (Si/AI=15) in benzene solvent. (Conditions: 400 mg n-propylphenol, 4 ml benzene, 40 mg catalyst, 2h reaction time)
Figure 5. Influence of solvent on product distributions over HZSM-5 (Si/AI=15) at 350 °C for 2h. (Conditions: 400 mg n-propylphenol, 4 ml solvent, 40 mg catalyst, 2h reaction time)
Figure 6. Catalytic dealkylation of n-propylphenol into phenol over the HZSM-5 (Si/AI=20) catalyst in benzene solvent using fixed-bed reactor. (Conditions: 200 mg HZSM-5, 10 wt% feedstock in benzene, feeding rate: 2.3 ml/h, pressure 50 bar)
Figure 7. Influence of support on the catalytic performance of Au-based catalysts during the demethoxylation of 2-methoxy-4-propylphenol at 350 °C for 2 h. (Conditions: 3000 mg feedstock, 30 ml benzene, 100 mg catalyst, 50 bar H₂, 2 h reaction time)

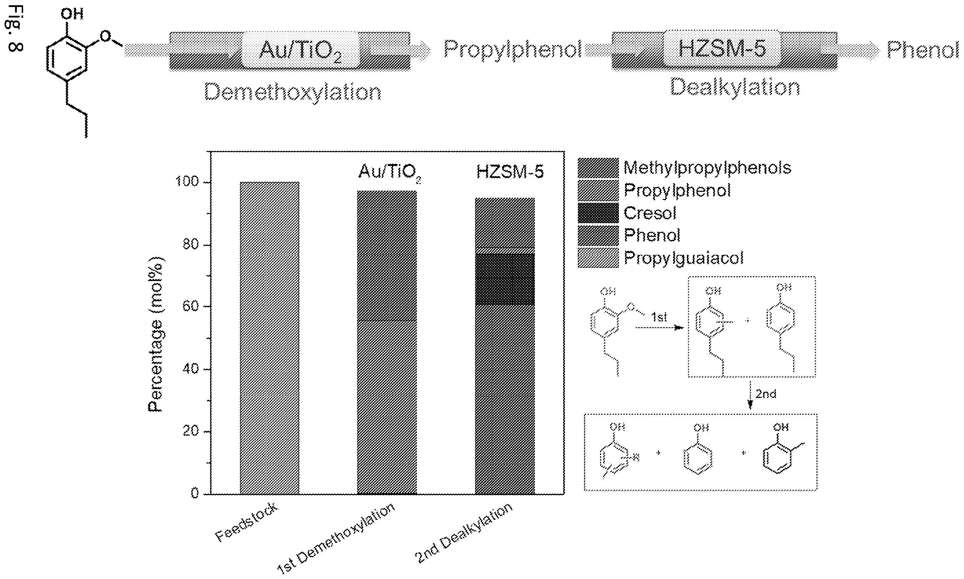

Figure 8. Two-step process for demethoxylation over 1 wt% Au/TiO₂ catalyst, followed by dealkylation into phenol over the HZSM-5 (Si/AI=15) catalyst in benzene solvent using fixed-bed reactor. (Conditions: 200 mg catalyst, 5 mol% 2-methoxy-4-propylphenol in benzene, feeding rate: 4.5 ml/h, pressure 100 bar, 30 ml/min H₂; the feedstock for the 2nd is obtained from the 1st demethoxylation step)

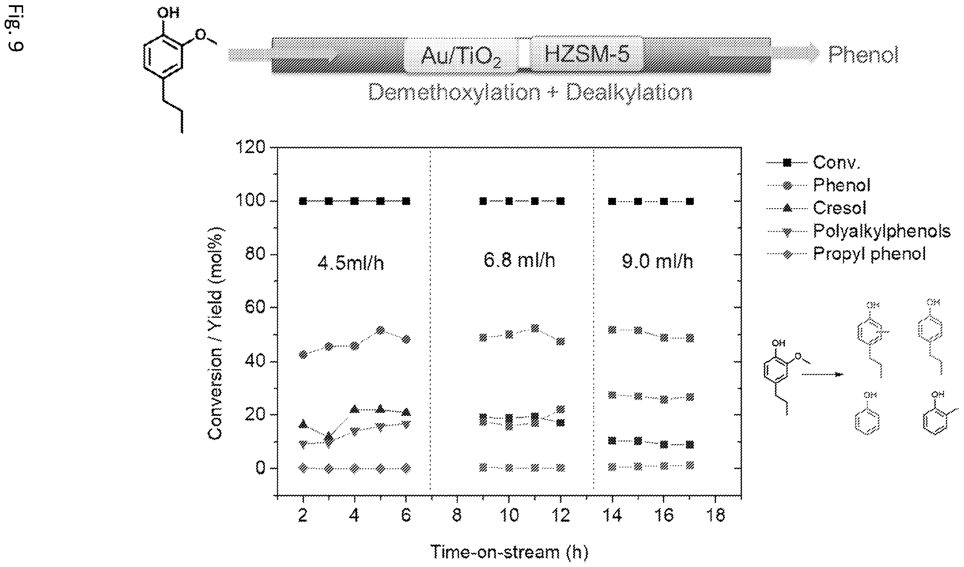

Figure 9. One-step process for demethoxylation and dealkylation over 1 wt% Au/TiO₂ and HZSM-5 (Si/AI=15) catalysts in benzene solvent using fixed-bed reactor. (Conditions: 100 mg Au/TiO₂ + 200 mg HZSM-5 catalysts, 5 mol% 2-methoxy-4-propylphenol in benzene, pressure 100 bar, 30 ml/min H₂)

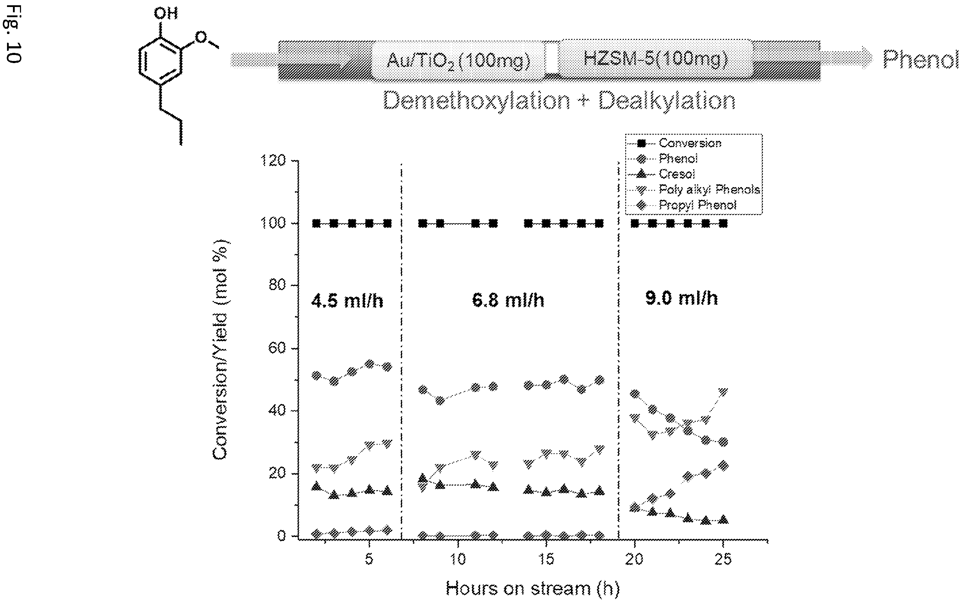

Figure 10. One-step process for demethoxylation and dealkylation over 1 wt% Au/TiO₂ and HZSM-5 (Si/AI=15) catalysts in benzene solvent using fixed-bed reactor. (Conditions: 100 mg Au/TiO₂ + 100 mg HZSM-5 catalysts, 5 mol% 2-methoxy-4-propylphenol in benzene, pressure 100 bar, 30 ml/min H₂)

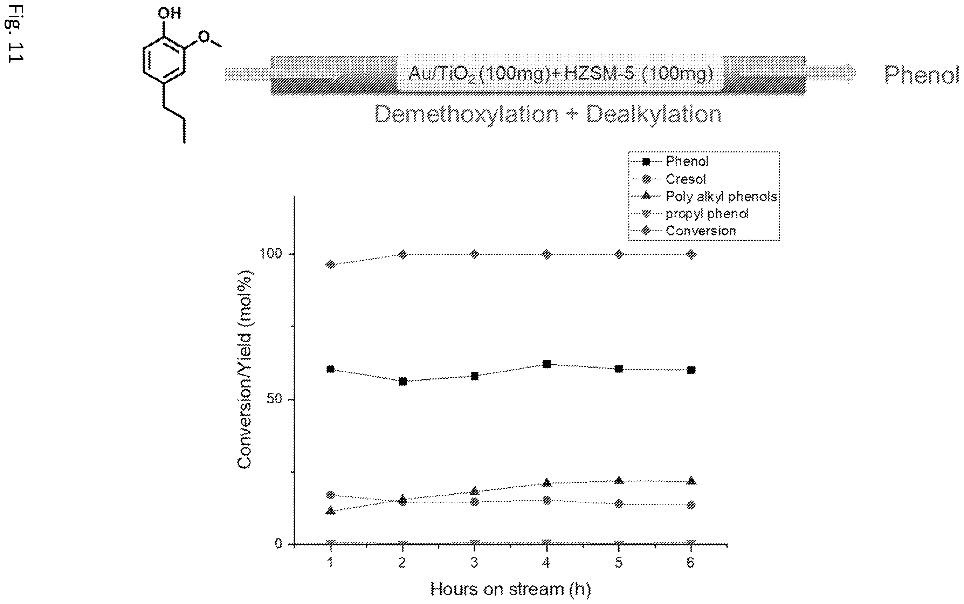

Figure 11. One-step process for demethoxylation and dealkylation over physical mixed 1 wt% Au/TiO₂ and HZSM-5 (Si/AI=15) catalysts in benzene solvent using fixed-bed reactor. (Conditions: 100 mg Au/TiO₂ + 100 mg HZSM-5 catalysts, 5 mol% 2-methoxy-4-propylphenol in benzene, feeding rate: 4.5ml/h, pressure 100 bar, 30 ml/min H₂)

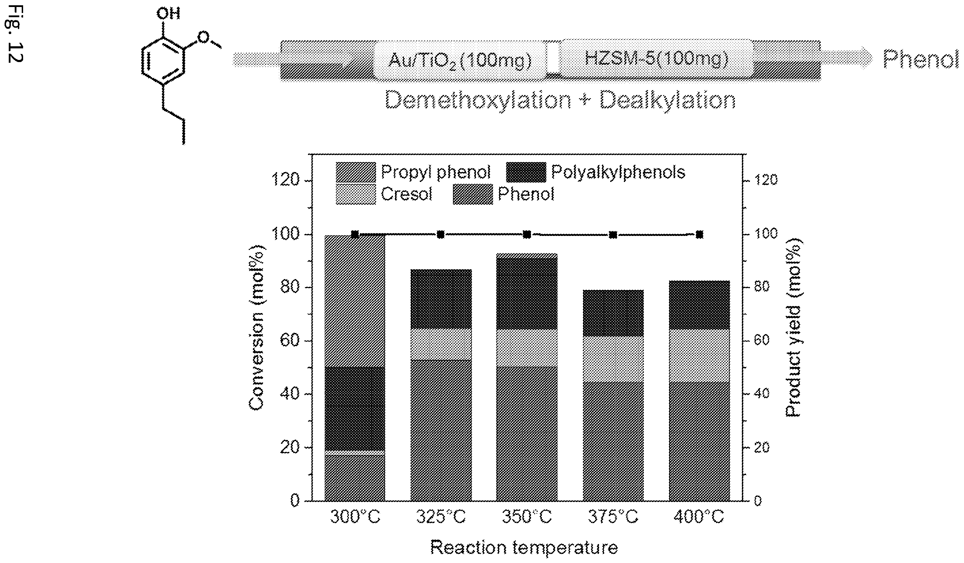

Figure 12. One-step process for demethoxylation and dealkylation over 1 wt% Au/TiO₂ and HZSM-5 (Si/AI=15) catalysts in benzene solvent using fixed-bed reactor at different temperature. (Conditions: 100 mg Au/TiO₂ + 100 mg HZSM-5 catalysts, 5 mol% 2-methoxy-4-propylphenol in benzene, feeding rate: 4.5ml/h, pressure 100 bar, 30 ml/min H₂)
Figure 13. One-step process for demethoxylation and dealkylation over stacked 1 wt% Au/TiO₂ and HZSM-5 (Si/AI=15) catalysts in benzene solvent using fixed-bed reactor at different H₂ pressure. (Conditions: 100 mg Au/TiO₂, 100 mg HZSM-5 catalyst, 6,8 ml/h)
Figure 14. Catalytic demethoxylation of 2-methoxy-4-propylphenol over (a) FeP/SiO₂, (b) Ni₂P/SiO₂, (c) CoP/SiO₂ , (d) WP/SiO₂ and (e) MoP/SiO₂ catalysts in benzene solvent: the influence on PG conversion and product distribution (Conditions: temperature 350 °C, 5.0 mol% 2-methoxy-4-propylphenol (feedstock) in benzene, feeding rate: 6.8 ml/h, WHSV=40 h⁻¹, H₂ pressure: 90bar).
Figure 15. Catalytic demethoxylation of 2-methoxy-4-propylphenol over (a) FeP/SiO₂, (b) Ni₂P/SiO₂, (c) CoP/SiO₂, (d) WP/SiO₂ and (e) MoP/SiO₂ catalysts in benzene solvent: the influence on benzene conversion and production distribution (Conditions: temperature 350 °C, 5.0 mol% 2-methoxy-4-propylphenol (feedstock) in benzene, feeding rate: 6.8 ml/h, WHSV=40 h⁻¹, H₂ pressure: 90bar).
Figure 16. One-step process for demethoxylation and transalkylation over MoP/HZSM-5 catalysts in benzene solvent: the influence of Mo loading (a) 0.4 mmol/g, (b) 0.8 mmol/g, (c) 1.2 mmol/g, (d) 1.6 mmol/g and (e) 2.0 mmol/g (Mo/P molar ratio 1) on PG conversion and product distribution (Conditions: temperature 350 °C, 5.0 mol% 2-methoxy-4-propylphenol (feedstock) in benzene, feeding rate: 6.8 ml/h, WHSV=40 h⁻¹, H₂ pressure: 90 bar).
Figure 17. One-step process for demethoxylation and transalkylation over MoP/HZSM-5 catalysts in benzene solvent: the influence of Mo loading (a) 0.4 mmol/g, (b) 0.8 mmol/g, (c)1.2 mmol/g, (d) 1.6 mmol/g and (e) 2.0 mmol/g (Mo/P molar ratio 1) on benzene conversion and product distribution (Conditions: temperature 350 °C, 5.0 mol% 2-methoxy-4-propylphenol (feedstock) in benzene, feeding rate: 6.8 ml/h, WHSV=40 h⁻¹, H₂ pressure: 90 bar).

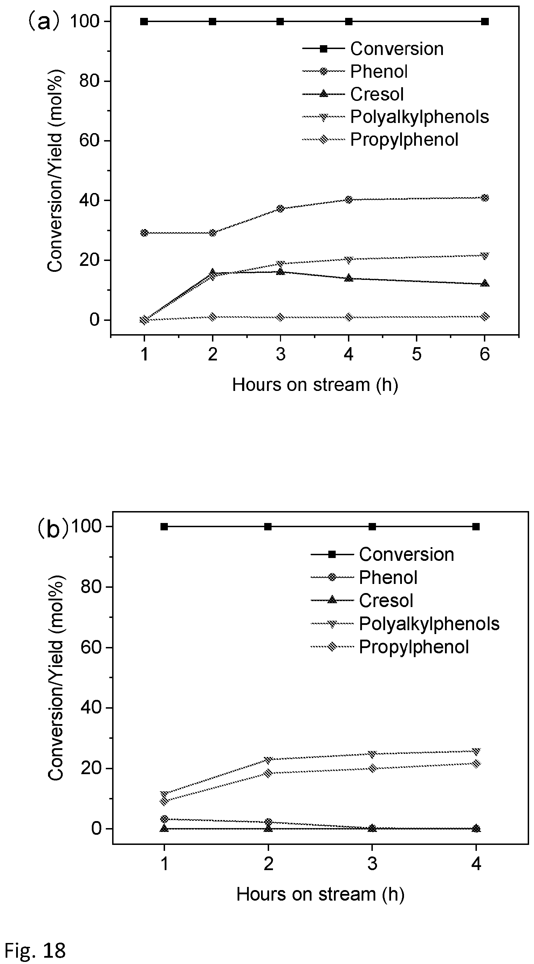

Figure 18. One-step process for demethoxylation and dealkylation over stacked 100mg 1 wt% Au/TiO₂ and 100mg HZSM-5 (Si/AI=15) catalysts in (a) toluene and (b) n-propylbenzene solvent using fixed-bed reactor. (Conditions: temperature: 350 °C, 5 mol% 2-methoxy-4-propylphenol, feeding rate: 6.8 ml/h, pressure 100 bar, 30 ml/min H₂)

### Examples

### Example 1: Dealkylation of propylphenol using mini-autoclave (Figure 1-5):

A mini-autoclave with a volume of 10 ml was used for the propylphenol dealkylation reactions. Typically, 40 mg of catalyst and 400 mg of feedstock were added to the reactor together with 4 ml solvent (e.g. benzene). After sealing, the autoclave is transferred in a GC oven which was preheated to the desired reaction temperature and maintained at that temperature for 2h. After reaction, the autoclave was removed from the oven and quenched in the ice bath. Then the reactor is opened and an amount of 10 ul dodecane was added to the reaction mixture as the internal standard. After separating the catalyst using membrane filtration or centrifugation, an aliquot of ca. 1ml of liquid sample was taken from the reaction mixture and directly subjected to GC-MS measurement for product identification and quantification.

### Example 2: Dealkylation of propylphenol using fixed bed reactor (Figure 6):

The reaction was performed using a home-built down-flow fixed-bed reactor (stainless steel, 6 mm inner diameter, 150 mm length). An amount of 200 mg HZSM-5 catalyst powder (125-250 µm) was loaded and immobilized in the tubular reactor. A solution of 10 wt% propylphenol in benzene was prepared and used as reaction substrate. Since external gas is not required, the reactor was pressurized to 50 bar by feeding the liquid feedstock using a HPLC pump. The reaction temperature was kept at 350 °C. When the reactor is stable, the propylphenol benzene solution was fed through a preheated (200 °C) feeding line using a HPLC pump at a set flow-rate of 0.05 ml/min (actual pumping rate: 2.3 ml/h). The liquid products were collected in a cold trap installed directly after the reactor. The samples were collected every 1 h. For each sample, an aliquot amount of 1 ml sample was accurately taken from the reaction mixture. After adding an amount of 10 µl dodecane internal standard, the sample was measured by GC-MS for product identification and quantification.

### Example 3: Demethoxylation of 2-methoxy-4-propylphenol using Parr autoclave (Figure 7):

A Parr autoclave with a volume of 100 ml was used for the 2-methoxy-4-propylphenol demethoxylation reactions. Typically, 100 mg of catalyst and 3000 mg of feedstock were added to the reactor together with 30 ml benzene solvent. After sealing, purging with H₂ and checking for leaks, the autoclave was brought to 50 bar by introducing H₂. Then the reactor was heated to 350 °C in ca. 60 min and maintained at that temperature for 2h. A small amount of sample was taken via a sampling valve after 1h and 2h reaction time. An aliquot amount of 1 ml sample was accurately taken from the reaction mixture. After adding an amount of 10 ul dodecane internal standard, the sample was measured by GC-MS for product identification and quantification. After reaction, the reactor was allowed to naturally cool down by removing the heating oven.

### Examp[e 4: Demethoxylation and transalkylation of 2-methoxy-4-propylphenol using fixed bed reactor (Figure 8-12 and Table 1):

A home-built down-flow fixed-bed reactor (stainless steel, 6 mm inner diameter, 150 mm length) was used for the dealkylation and demethoxylation reaction. In a typical experiment, 25-100 mg Au/TiO₂ (125-250 µm) and 100-200 mg HZSM-5 catalyst powder (125-250 µm) were loaded in the tubular reactor either in the stacked (Au/TiO₂ on top of HZSM-5) or homogeneously mixed way. For the stacked catalyst bed, a small amount of quartz wool was added in between to separate the two catalysts. The pressure was maintained at 100 bar by introducing H₂ with a controlled flow rate of 30 ml/min. Typically, the temperature was maintained at 350 °C. When the reactor is stable, the 2-methoxy-4-propylphenol benzene solution with concentration of 5 mol% was fed through a preheated (200 °C) feeding line using a HPLC pump at a set flow-rate of 0.05-0.2 ml/min (actual pumping rate: 2.3-9.0 ml/h). The liquid products were collected in a cold trap installed directly after the reactor. The samples were collected every 1 h. For each sample, an aliquot amount of 1 ml sample was accurately taken from the reaction mixture. After adding an amount of 10 µl dodecane internal standard, the sample was measured by GC-MS for product identification and quantification.

**Table 1. One-step process for demethoxylation and dealkylation over physical mixed or stacked 1 wt% Au/TiO2 and HZSM-5 (Si/Al=15) catalysts in benzene solvent using fixed-bed reactor. (Conditions: temperature: 350 °C, 5 mol% 2-methoxy-4-propylphenol (feedstock) in benzene, feeding rate: 6.8 ml/h, pressure 100 bar, 30 ml/min H2)**

| **Catalyst bed** | **Amount of catalyst (mg)** | | **Hours** | **Conversion** | **Yield (mol%)** | | | |
|---|---|---|---|---|---|---|---|---|
| | **Au/TiO₂** | **HZSM-5** | | | **Phenol** | **Cresol** | **Polyalkylphenols** | **Propylphenol** |
| Mixed bed | 50 | 100 | 1 | 91.6 | 47.8 | 9.4 | 9.7 | 0.8 |
| | | | 2 | 99.8 | 53.4 | 9.6 | 12.1 | 0.4 |
| | | | 3 | 100.0 | 61.8 | 10.5 | 18.5 | 0.6 |
| | | | 4 | 100.0 | 57.4 | 7.7 | 22.7 | 1.4 |
| | | | 5 | 100.0 | 57.7 | 9.1 | 22.2 | 1.8 |
| | | | 6 | 100.0 | 57.4 | 8.4 | 22.8 | 3.0 |
| Mixed bed | 100 | 100 | 1 | 96.28 | 57.6 | 15.2 | 12.2 | 0.6 |
| | | | 2 | 100.00 | 53.6 | 13.2 | 15.9 | 0.4 |
| | | | 3 | 100.00 | 55.3 | 13.1 | 18.8 | 0.5 |
| | | | 4 | 100.00 | 59.2 | 13.6 | 21.7 | 0.6 |
| | | | 5 | 100.00 | 57.7 | 12.6 | 22.3 | 0.4 |
| | | | 6 | 100.00 | 57.3 | 12.2 | 22.3 | 0.6 |
| Stacked bed | 100 | 100 | 1 | 100.0 | 48.3 | 14.1 | 21.4 | 0.3 |
| | | | 2 | 100.0 | 48.2 | 14.7 | 23.3 | 0.0 |
| | | | 3 | 100.0 | 48.4 | 14.0 | 26.5 | 0.4 |
| | | | 4 | 100.0 | 50.1 | 15.1 | 26.4 | 0.0 |
| | | | 5 | 100.0 | 46.9 | 13.5 | 24.0 | 0.4 |
| | | | 6 | 100.0 | 49.9 | 14.4 | 28.0 | 0.2 |
| Stacked bed | 50 | 100 | 1 | 69.9 | 36.1 | 6.8 | 16.6 | 0.0 |
| | | | 2 | 99.5 | 46.4 | 8.2 | 22.5 | 0.0 |
| | | | 3 | 97.5 | 35.8 | 6.1 | 26.2 | 0.1 |
| | | | 4 | 99.3 | 41.5 | 7.0 | 28.9 | 0.1 |
| | | | 5 | 98.8 | 35.2 | 5.7 | 32.3 | 0.0 |
| | | | 6 | 98.4 | 32.4 | 5.3 | 39.1 | 0.0 |
| Stacked bed | 25 | 100 | 1 | 94.0 | 16.1 | 3.9 | 9.1 | 4.1 |
| | | | 2 | 86.9 | 11.5 | 3.6 | 21.6 | 12.4 |
| | | | 3 | 74.7 | 7.2 | 2.4 | 22.3 | 10.7 |
| | | | 4 | 84.1 | 2.4 | 0.3 | 33.8 | 21.1 |
| | | | 5 | 59.3 | 0.8 | 0.0 | 27.9 | 19.1 |
| | | | 6 | 50.7 | 0.3 | 0.0 | 27.3 | 20.5 |

### Example 5: Preparation of Metal phosphide catalysts:

Transition metal phosphide catalysts are prepared by the temperature-programmed reduction method. The metal loading is 1.6 mmol/g of support. The metal/phosphorus molar ratio is ½ for Ni₂P and FeP, and 1 for MoP, CoP and WP. The precursors (Ni(NO₃)₂·6H₂O, Fe(NO₃)₃·9H₂O, (NH4)₆Mo₇O₂₄·4H₂O, Co(NO₃)₂·6H₂O, (NH₄)₆W₁₂O₃₉·xH₂O) were dissolved in pure water and the solutions were used to impregnate the silica support by incipient wetness impregnation. The obtained catalysts were dried at 105 °C overnight and calcined at 500 °C for 6 h. The phosphorus precursor solution ((NH₄)₂HPO₄) was dropped into the obtained catalyst by incipient wetness impregnation. After dried at 105 °C overnight, the obtained catalysts were grounded and sieved to a particle size of 75-200 µm. The sieved catalysts were loaded in a quartz tube for reduction. The hydrogen flow rate was 100 ml/min. The temperature was increased to 700 °C with a ramping rate of 3 °C/min. The reduction time was 3 hours. After reduction, the oven was cooled down to room temperature. The obtained catalyst was passivated at room temperature in a 0.5% O₂ in Ar for 2 hours.

A set of supported transition metal phosphide catalysts including FeP/SiO₂, Ni₂P/SiO₂, CoP/SiO₂, WP/SiO₂ and MoP/SiO₂ were synthesized and tested for demethoxylation of 2-methoxy-4-propylphenol in benzene solvent. The reactions were carried out in the fixed-bed reactor at 350°C and 90 bar H₂. The experimental procedure is similar to the process of demethoxylation and transalkylation using fixed bed reactor involving the Au/TiO₂ and HZSM-5. Among the tested catalysts, MoP/SiO₂ is the most active catalyst for demethoxylation, delivering about 90% yield of propylphenol and 10% methylated propylphenol at 100% conversion (Figure 14e). Notably, only a small amount of toluene was produced by benzene methylation. The Ni₂P/SiO₂ also showed high activity towards deoxygenation and ring hydrogenation. The main products are propylcyclohexane and propylbenzene (Figure 14b). In this case, the benzene-derived product is mainly cyclohexane (Figure 15b).

### Example 6: One-step demethoxylation and transalkylation of PG over MoP/HZSM-5 catalyst

The MoP/HZSM-5 was synthesized by wetness impregnation followed by reduction using H₂. The purpose was to test whether the demethoxylation and transalkylation processes can be catalyzed by one catalyst. The influence of Mo loading varying from 0.4 mmol/g to 2.0 mmol/g on catalytic performance were evaluated. The optimal Mo loading was found to be 0.8 mmol/g, yield about 60% yield of phenol at about 100% conversion (Figure 16).

### Example 7: One-step demethoxylation and transalkylation of 2-methoxy-4-propylphenol over MoP/SiO₂ and HZSM-5 catalysts

One-step demethoxylation and transalkylation of 2-methoxy-4-propylphenol was further tested using MoP/SiO₂ and HZSM-5 in benzene solvent at 350 °C. Stacked-bed catalyst configuration with MoP/SiO₂ on top of HZSM-5 was used in this set of experiments. The combination of these two catalysts delivered high activity for PG conversion. Up to 90% yield of phenol and 8% yield of cresol could be obtained at 100% conversion (Table 3). Notably, this high catalytic performance could be maintained within the 6 h time on stream. This suggests that the catalyst is stable within the tested period.

**Table 3. One-step process for demethoxylation and dealkylation over stacked MoP/SiO₂ and HZSM-5 (Si/Al=15) catalysts in benzene solvent using fixed-bed reactor. (Conditions: temperature: 350 °C, 5 mol% 2-methoxy-4-propylphenol in benzene, feeding rate: 6.8 ml/h, pressure 90 bar, 30 ml/min H₂).**

| **Catalyst bed** | **Amount of catalyst (mg)** | | **Hours** | **Conversion (%)** | **Yield (mol%)** | | |
|---|---|---|---|---|---|---|---|
| | **MoP/SiO₂** | **HZSM-5** | | | **Phenol** | **Cresol** | **2-Propyl phenol** |
| Stacked bed | 100 | 100 | 1 | 100.0 | 85.3 | 7.4 | 0.3 |
| | | | 2 | 100.0 | 89.5 | 7.4 | 0.2 |
| | | | 3 | 100.0 | 89.9 | 7.9 | 0.3 |
| | | | 4 | 100.0 | 91.9 | 8.1 | 0.4 |
| | | | 5 | 100.0 | 87.0 | 7.8 | 0.4 |
| | | | 6 | 100.0 | 90.8 | 8.3 | 0.4 |

## Claims

1. A process for converting alkylmethoxyphenols, wherein alkylmethoxyphenols are demethoxylated in the presence of an aromatic solvent and a first catalyst to form alkylphenols, wherein the first catalyst contains a metal selected from groups consisting of Group 5 (VB), Group 6 (VIB), Group 7 (VI IB), Group 8 (VIIIB), Group 9 (VIIIB), Group 10 (VIIIB), Group 11 (IB), Group 12 (IIB), Group 13 (IIIA), Group 14 (IVA), Group 15 (VA) and Group 16 (VIA), wherein the IUPAC group number appears first and an old system of group numbers appears in parenthesis, or any alloy or mixture thereof, combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon oxide, wherein the aromatic solvents are selected from the group consisting of benzene, toluene, ethylbenzene, propylbenzene or combinations thereof.

2. A process for converting alkylphenols, wherein alkylphenols are dealkylated in the presence of an aromatic solvent and a second catalyst to form phenol, wherein the second catalyst is a zeolite or zeotype material, wherein the aromatic solvents are selected from the group consisting of benzene, toluene, ethylbenzene, propylbenzene or combinations thereof.

3. Process according to claim 1, wherein the process further comprises a dealkylation process according to claim 2.

4. Process according to claim 3, wherein the demethoxylation process and the dealkylation process take place in the same reactor, preferably wherein the processes are performed in a continuous fixed bed flow reactor or a batch reactor.

5. Process according to any one of the preceding claims, wherein the demethoxylation and/or the dealkylation process is performed at a temperature between 200 to 500 °C, preferably 250 - 450 °C, more preferably 300 - 400 °C most preferably around 350 °C.

6. Process according to any one of the preceding claims, wherein the demethoxylation and/or the dealkylation process is performed at a pressure of 1 - 250 bar, preferably 10 - 200 bar, more preferably 50 - 180 bar, most preferably 80 - 150 bar.

7. Process according to any one of the preceding claims, wherein the demethoxylation and/or the dealkylation process is performed such that the residence time in the reactor is between 0.5 and 24 hours, preferably between 1 and 18 hours, more preferably between 2 and 12 hours, even more preferably between 4 and 10 hours, most preferably at least 2 hours.

8. Process according to any one of the preceding claims, wherein the aromatic solvent is at least 50% v/v benzene, more preferably at least 80% v/v benzene, even more preferably at least 90% v/v benzene, even more preferably at least 95% v/v most preferably 99% v/v benzene.

9. Process according to any one of claims 2 to 8, wherein the second catalyst used in the dealkylation process is a zeolite or zeotype material, preferably . wherein the zeolite or zeotype material is **characterized by**:
(i) acid sites of sufficient strength, preferably being aluminosilicates or silicates with metal and/or metalloid substitutions, more preferably silicates with Fe, Ga and/or B substitutions, and
(ii) having a pore structure ranging from 3 - 13 Angstrom, preferable having a pore structure with ranges identified for 10 membered ring zeolites.

10. Process according to any one of claims 2 to 9, wherein the second catalyst used in the dealkylation process comprises aluminosilicates, preferably wherein the second catalyst is HZSM-5.

11. Process according to any one of claims 1 - 10, wherein the first catalyst comprises gold combined with a support selected from the group consisting of activated carbon, mesoporous carbon, aluminum oxide, zeolites, titanium oxide, cerium oxide, molybdenum oxide, zinc oxide, iron oxide, vanadium, zirconium oxide and silicon dioxide, preferably wherein the first catalyst is Au/TiO₂.

12. Process according to any one of claims 1 - 10, wherein the first catalyst is a SiO₂ supported metal phosphide catalyst, preferably wherein the first catalyst is selected from the group consisting of CoP/SiO₂, WP/SiO₂ and MoP/SiO₂, more preferably wherein the first catalyst is MoP/SiO₂, most preferably wherein the first catalyst is MoP/SiO₂ and the catalyst has a Mo loading of 1.6 mmol/g or less.

13. Process according to any one of claims 3 - 12, wherein the first and the second catalyst are either stacked or homogeneously mixed with each other.

14. Process according to any of the preceding claims, wherein the alkylmethoxyphenols and/or the alkylphenols are lignin-derived, preferably the alkylmethoxyphenols are obtained from depolymerisation of lignin.

## Patentansprüche

1. Verfahren zum Umwandeln von Alkylmethoxyphenolen, wobei Alkylmethoxyphenole in der Gegenwart eines aromatischen Lösungsmittels und eines ersten Katalysators demethoxyliert werden, um Alkylphenole auszubilden, wobei der erste Katalysator ein Metall, das aus Gruppen ausgewählt ist, bestehend aus Gruppe 5 (VB), Gruppe 6 (VIB), Gruppe 7 (VIIB), Gruppe 8 (VIIIB), Gruppe 9 (VIIIB), Gruppe 10 (VIIIB), Gruppe 11 (IB), Gruppe 12 (IIB), Gruppe 13 (IIIA), Gruppe 14 (IVA), Gruppe 15 (VA) und Gruppe 16 (VIA), wobei die IUPAC-Gruppennummer zuerst erscheint und ein altes System von Gruppennummern in Klammern erscheint, oder eine beliebige Legierung oder Mischung davon enthält, kombiniert mit einem Träger, der aus der Gruppe ausgewählt ist, bestehend aus aktiviertem Kohlenstoff, mesoporösem Kohlenstoff, Aluminiumoxid, Zeolithen, Titanoxid, Ceroxid, Molybdänoxid, Zinkoxid, Eisenoxid, Vanadium, Zirconiumoxid und Siliciumoxid, wobei die aromatischen Lösungsmittel aus der Gruppe ausgewählt sind, bestehend aus Benzol, Toluol, Ethylbenzol, Propylbenzol oder Kombinationen davon.

2. Verfahren zum Umwandeln von Alkylphenolen, wobei Alkylphenole in der Gegenwart eines aromatischen Lösungsmittels und eines zweiten Katalysators dealkyliert werden, um Phenol auszubilden, wobei der zweite Katalysator ein Zeolith- oder Zeotypmaterial ist, wobei die aromatischen Lösungsmittel aus der Gruppe ausgewählt sind, bestehend aus Benzol, Toluol, Ethylbenzol, Propylbenzol oder Kombinationen davon.

3. Verfahren nach Anspruch 1, wobei das Verfahren ferner ein Dealkylierungsverfahren nach Anspruch 2 umfasst.

4. Verfahren nach Anspruch 3, wobei das Demethoxylierungsverfahren und das Dealkylierungsverfahren in dem gleichen Reaktor stattfinden, wobei vorzugsweise die Verfahren in einem kontinuierlichen Festbettdurchflussreaktor oder einem Satzreaktor durchgeführt werden.

5. Verfahren nach einem der vorstehenden Ansprüche, wobei das Demethoxylierungs- und/oder das Dealkylierungsverfahren bei einer Temperatur von zwischen 200 und 500 °C, vorzugsweise 250-450 °C, mehr bevorzugt 300-400 °C, am meisten bevorzugt ungefähr 350 °C, durchgeführt wird.

6. Verfahren nach einem der vorstehenden Ansprüche, wobei das Demethoxylierungs- und/oder das Dealkylierungsverfahren bei einem Druck von 1-250 bar, vorzugsweise 10-200 bar, mehr bevorzugt 50-180 bar, am meisten bevorzugt 80-150 bar, durchgeführt wird.

7. Verfahren nach einem der vorstehenden Ansprüche, wobei das Demethoxylierungs- und/oder das Dealkylierungsverfahren derart durchgeführt wird, dass die Verweildauer in dem Reaktor zwischen 0,5 und 24 Stunden, vorzugsweise zwischen 1 und 18 Stunden, mehr bevorzugt zwischen 2 und 12 Stunden, noch mehr bevorzugt zwischen 4 und 10 Stunden, am meisten bevorzugt mindestens 2 Stunden, beträgt.

8. Verfahren nach einem der vorstehenden Ansprüche, wobei das aromatische Lösungsmittel zu mindestens 50 Vol.-% Benzol, mehr bevorzugt zu mindestens 80 Vol.-% Benzol, noch mehr bevorzugt zu mindestens 90 Vol.-% Benzol, noch mehr bevorzugt zu mindestens 95 Vol.-%, am meisten bevorzugt zu 99 Vol.-% Benzol, ist.

9. Verfahren nach einem der Ansprüche 2 bis 8, wobei der zweite Katalysator, der in dem Dealkylierungsverfahren verwendet wird, ein Zeolith- oder Zeotypmaterial ist, wobei vorzugsweise das Zeolith- oder Zeotypmaterial **gekennzeichnet ist durch:**
(i) Säurestellen ausreichender Stärke, die vorzugsweise Aluminosilicate oder Silicate mit Metall- und/oder Halbmetallsubstitutionen, mehr bevorzugt Silicate mit Fe-, Ga- und/oder B-Substitutionen, sind und
(ii) Aufweisen einer Porenstruktur, die von 3-13 Angström reicht, vorzugsweise Aufweisen einer Porenstruktur mit Bereichen, die für 10-gliedrige Ringzeolithen identifiziert sind.

10. Verfahren nach einem der Ansprüche 2 bis 9, wobei der zweite Katalysator, der in dem Dealkylierungsverfahren verwendet wird, Aluminosilicate umfasst, wobei vorzugsweise der zweite Katalysator HZSM-5 ist.

11. Verfahren nach einem der Ansprüche 1 bis 10, wobei der erste Katalysator Gold kombiniert mit einem Träger umfasst, der aus der Gruppe ausgewählt ist, bestehend aus aktiviertem Kohlenstoff, mesoporösem Kohlenstoff, Aluminiumoxid, Zeolithen, Titanoxid, Ceroxid, Molybdänoxid, Zinkoxid, Eisenoxid, Vanadium, Zirconiumoxid und Siliciumdioxid, wobei vorzugsweise der erste Katalysator Au/TiO₂ ist.

12. Verfahren nach einem der Ansprüche 1 bis 10, wobei der erste Katalysator ein SiO₂-Trägermetallphosphidkatalysator ist, wobei vorzugsweise der erste Katalysator aus der Gruppe ausgewählt ist, bestehend aus CoP/SiO₂, WP/SiO₂ und MoP/SiO₂, wobei mehr bevorzugt der erste Katalysator MoP/SiO₂ ist, wobei am meisten bevorzugt der erste Katalysator MoP/SiO₂ ist und der Katalysator eine Mo-Beladung von 1,6 mmol/g oder weniger aufweist.

13. Verfahren nach einem der Ansprüche 3 bis 12, wobei der erste und der zweite Katalysator entweder gestapelt oder homogen miteinander vermischt sind.

14. Verfahren nach einem der vorstehenden Ansprüche, wobei die Alkylmethoxyphenole und/oder die Alkylphenole von Lignin abgeleitet sind, wobei vorzugsweise die Alkylmethoxyphenole durch Depolymerisation von Lignin erhalten werden.

## Revendications

1. Procédé permettant de convertir des alkylméthoxyphénols, dans lequel des alkylméthoxyphénols sont déméthoxylés en présence d'un solvant aromatique et d'un premier catalyseur pour former des alkylphénols, dans lequel le premier catalyseur contient un métal choisi parmi des groupes constitués par Groupe 5 (VB), Groupe 6 (VIB), Groupe 7 (VIIB), Groupe 8 (VIIIB), Groupe 9 (VIIIB), Groupe 10 (VIIIB), Groupe 11 (IB), Groupe 12 (IIB), Groupe 13 (IIIA), Groupe 14 (IVA), Groupe 15 (VA) et Groupe 16 (VIA), dans lequel le numéro de groupe UICPA apparaît en premier et un ancien système de numéros de groupe apparaît entre parenthèses, ou l'un quelconque alliage ou mélange de ceux-ci, combiné avec un support choisi dans le groupe constitué par charbon actif, carbone mésoporeux, oxyde d'aluminium, zéolites, oxyde de titane, oxyde de cérium, oxyde de molybdène, oxyde de zinc, oxyde de fer, vanadium, oxyde de zirconium et oxyde de silicium, dans lequel les solvants aromatiques sont choisis dans le groupe constitué par benzène, toluène, éthylbenzène, propylbenzène ou combinaisons de ceux-ci.

2. Procédé permettant de convertir des alkylphénols, dans lequel des alkylphénols sont désalkylés en présence d'un solvant aromatique et d'un second catalyseur pour former du phénol, dans lequel le second catalyseur est une zéolite ou un matériau zéotype, dans lequel les solvants aromatiques sont choisis dans le groupe constitué par benzène, toluène, éthylbenzène, propylbenzène ou combinaisons de ceux-ci.

3. Procédé selon la revendication 1, dans lequel le procédé comprend en outre un procédé de désalkylation selon la revendication 2.

4. Procédé selon la revendication 3, dans lequel le procédé de déméthoxylation et le procédé de désalkylation ont lieu dans le même réacteur, de préférence dans lequel les procédés sont mis en oeuvre dans un réacteur continu à écoulement à lit fixe ou un réacteur discontinu.

5. Procédé selon l'une quelconque des revendications précédentes, dans lequel le procédé de déméthoxylation et/ou de désalkylation est mis en oeuvre à une température comprise de 200 à 500 °C, de préférence 250 à 450 °C, plus préférablement 300 à 400 °C le plus préférablement d'environ 350 °C.

6. Procédé selon l'une quelconque des revendications précédentes, dans lequel le procédé de déméthoxylation et/ou de désalkylation est mis en oeuvre à une pression de 1 à 250 bar, de préférence 10 à 200 bar, plus préférablement 50 à 180 bar, le plus préférablement 80 à 150 bar.

7. Procédé selon l'une quelconque des revendications précédentes, dans lequel le procédé de déméthoxylation et/ou de désalkylation est mis en oeuvre de telle sorte que le temps de séjour dans le réacteur est compris entre 0,5 et 24 heures, de préférence entre 1 et 18 heures, plus préférablement entre 2 et 12 heures, même plus préférablement entre 4 et 10 heures, le plus préférablement est d'au moins 2 heures.

8. Procédé selon l'une quelconque des revendications précédentes, dans lequel le solvant aromatique est au moins 50 % en volume de benzène, plus préférablement au moins 80 % en volume de benzène, encore plus préférablement au moins 90 % en volume de benzène, encore plus préférablement au moins 95 % en volume le plus préférablement 99 % en volume de benzène.

9. Procédé selon l'une quelconque des revendications 2 à 8, dans lequel le second catalyseur utilisé dans le procédé de désalkylation est une zéolite ou un matériau zéotype, de préférence dans lequel la zéolite ou le matériau zéotype est **caractérisé par** :
(i) des sites acides de force suffisante, étant de préférence des aluminosilicates ou silicates avec substitutions de métal et/ou de métalloïde, plus préférablement des silicates avec des substitutions Fe, Ga et/ou B, et
(ii) ayant une structure de pores allant de 3 à 13 Angströms, de préférence ayant une structure de pores avec des plages identifiées pour des zéolites cycliques à 10 chaînons.

10. Procédé selon l'une quelconque des revendications 2 à 9, dans lequel le second catalyseur utilisé dans le procédé de désalkylation comprend des aluminosilicates, de préférence dans lequel le second catalyseur est HZSM-5.

11. Procédé selon l'une quelconque des revendications 1 à 10, dans lequel le premier catalyseur comprend de l'or combiné avec un support choisi dans le groupe constitué par charbon actif, carbone mésoporeux, oxyde d'aluminium, zéolites, oxyde de titane, oxyde de cérium, oxyde de molybdène, oxyde de zinc, oxyde de fer, vanadium, oxyde de zirconium et dioxyde de silicium, de préférence dans lequel le premier catalyseur est Au/TiO₂.

12. Procédé selon l'une quelconque des revendications 1 à 10, dans lequel le premier catalyseur est un catalyseur phosphure de métal supporté sur SiO₂, de préférence dans lequel le premier catalyseur est choisi dans le groupe constitué par CoP/SiO₂, WP/SiO₂ et MoP/SiO₂, plus préférablement dans lequel le premier catalyseur est MoP/SiO₂, le plus préférablement dans lequel le premier catalyseur est MoP/SiO₂ et le catalyseur a un chargement en Mo de 1,6 mmol/g ou moins.

13. Procédé selon l'une quelconque des revendications 3 à 12, dans lequel le premier et le second catalyseur sont soit empilés soit mélangés de façon homogène l'un à l'autre.

14. Procédé selon l'une quelconque des revendications précédentes, dans lequel les alkylméthoxyphénols et/ou les alkylphénols sont dérivés de lignine, de préférence les alkylméthoxyphénols sont obtenus par dépolymérisation de lignine.
